(19)

Europäisches
Patentamt
European
Patent Office
Office européen
des brevets

(11)  **EP 2 150 811 B1**

(12)  **EUROPEAN PATENT SPECIFICATION**

(45) Date of publication and mention
of the grant of the patent:
**16.01.2013 Bulletin 2013/03**

(51) Int Cl.:
***G01N 33/68*** *(2006.01)*

(21) Application number: **08769335.4**

(22) Date of filing: **08.05.2008**

(86) International application number:
**PCT/US2008/063070**

(87) International publication number:
**WO 2008/137991 (13.11.2008 Gazette 2008/46)**

(54) **HUMAN B-TYPE NATRIURETIC PEPTIDE ASSAY HAVING REDUCED CROSS-REACTIVITY WITH OTHER PEPTIDE FORMS**

TEST FÜR MENSCHLICHES NATRIURETISCHES TYP-B-PEPTID MIT VERMINDERTER KREUZREAKTIVITÄT MIT ANDEREN PEPTIDFORMEN

DOSAGE DE PEPTIDE NATRIURÉTIQUE DE TYPE B HUMAIN AYANT UNE RÉACTIVITÉ CROISÉE RÉDUITE AVEC D'AUTRES FORMES PEPTIDIQUES

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **08.05.2007 US 916718 P**

(43) Date of publication of application:
**10.02.2010 Bulletin 2010/06**

(73) Proprietor: **Abbott Laboratories**
**Abbott Park, Illinois 60064-6008 (US)**

(72) Inventors:
• **MOORE, Jeffrey A.**
**Gurnee, Illinois 60031 (US)**
• **SHIH, Jessie**
**Lake Forest, Illinois 60045 (US)**

(74) Representative: **Modiano, Micaela Nadia et al**
**Modiano & Partners**
**Thierschstrasse 11**
**80538 München (DE)**

(56) References cited:
**US-A1- 2004 265 926    US-A1- 2006 183 154**
**US-A1- 2006 183 154    US-A1- 2007 207 152**

• **LAM C S P ET AL: "Alternate Circulating Pro-B-Type Natriuretic Peptide and B-Type Natriuretic Peptide Forms in the General Population" JOURNAL OF THE AMERICAN COLLEGE OF CARDIOLOGY, ELSEVIER, NEW YORK, NY, US LNKD- DOI:10.1016/J.JACC.2006.12.024, vol. 49, no. 11, 20 March 2007 (2007-03-20), pages 1193-1202, XP022666242 ISSN: 0735-1097 [retrieved on 2007-03-14]**
• **GOETZE J P ET AL: "Acute myocardial hypoxia increases BNP gene expression." THE FASEB JOURNAL : OFFICIAL PUBLICATION OF THE FEDERATION OF AMERICAN SOCIETIES FOR EXPERIMENTAL BIOLOGY DEC 2004 LNKD-PUBMED:15576492, vol. 18, no. 15, December 2004 (2004-12), pages 1-18, XP002605802 ISSN: 1530-6860**
• **HUNT P J ET AL: "THE AMINO-TERMINAL PORTION OF PRO-BRAIN NATRIURETIC PEPTIDE (PRO-BNP) CIRCULATES IN HUMAN PLASMA" BIOCHEMICAL AND BIOPHYSICAL RESEARCH COMMUNICATIONS, ACADEMIC PRESS INC. ORLANDO, FL, US LNKD- DOI: 10.1006/BBRC.1995.2410, vol. 214, no. 3, 1 January 1995 (1995-01-01), pages 1175-1183, XP000907386 ISSN: 0006-291X**
• **LABORATORY REFERENCE MANUAL: 'Calculations' REED COLLEGE, [Online] 21 February 2007, XP008135649 Retrieved from the Internet: <URL:http://www.web.archive.org/web/2007022 1044313/http: //www.academic.reed.edu/chemis try/alan/ 201_202/ab_manual/appendices/calcu lations.html>**

EP 2 150 811 B1

**(Cont. next page)**

- **TSUTAMOTO T. ET AL.: 'Attenuation of Compensation of Endogenous Cardiac Natriuretic Peptide System in Chronic Heart Failure' CIRCULATION vol. 96, 1997, pages 509 - 516, XP009092327**

- **COLUCCI W.S. ET AL.: 'Intravenous nesiritide, a natriuretic peptide, in the treatment of decompensated congestive heart failure' N. ENGL. MED. vol. 343, no. 4, 2005, pages 246 - 253, XP009033850**

**Description**

TECHNICAL FIELD

The present disclosure relates to assays for detecting and/or quantifying the amount of human B-type natriuretic peptide in a test sample. Specifically, the assays of the present disclosure exhibit less than about twenty percent (20%) cross-reactivity with any human proB-type natriuretic peptide present or contained in a test sample.

BACKGROUND

Atrial natriuretic peptide (hereinafter "ANP"), B-type natriuretic peptide (hereinafter "BNP"), C-type natriuretic peptide (hereinafter "CNP") and Dendroaspis natriuretic peptide (hereinafter "DNP") are each members of a family of hormones known as "natriuretic peptides". ANP and BNP share a wide spectrum of biological properties and belong to the cardiac natriuretic system. Both ANP and BNP are of myocardial cell origin while CNP is of endothelial cell origin. DNP was isolated from the venom of the green mamba snake and possesses structural similarity to ANP, BNP and CNP.

ANP is secreted by the heart in the atria. ANP has a 17 amino acid ring closed by a disulfide bond between two cysteine residues. Eleven of the seventeen amino acids in the ring are conserved across ANP, BNP, CNP and DNP. In addition to the 17 amino acid ring structure, ANP has an amino-terminal tail of 6 amino acids and a carboxy-terminal tail of 5 amino acids. ANP is produced as a 126 amino acid pro-ANP form that is the major storage form of ANP. After proteolytic cleavage between amino acids 98 and 99, the mature 28 amino acid peptide ANP is found in coronary sinus plasma (See Yandle, J. Internal Med., 235:561-576 (1994)).

BNP received its name because it was first isolated from porcine brain, thus, initially, "BNP" stood for "brain natriuretic peptide". However, because BNP was found to belong to the cardiac natriuretic system, the word "brain" was changed to "B-type". Therefore, "BNP" now refers to "B-type natriuretic peptide". In humans, BNP is secreted by the heart through the coronary sinus, predominantly from the cardiac ventricles. The pre-pro peptide precursor of human BNP (hereinafter "human pre-proBNP") is 134 amino acids in length (SEQ ID NO:1) and comprises a short signal peptide, which is enzymatically cleaved off to release the human pro peptide of BNP (hereinafter "human proBNP") which is 108 amino acids in length (SEQ ID NO:2). Human proBNP is further cleaved into an N-terminal pro peptide of human BNP (hereinafter "human NT-proBNP") which is 76 amino acids in length (SEQ ID NO:3) and the active hormone, human BNP (hereinafter "hBNP" or "hBNP-32"), which is 32 amino acids in length (SEQ ID NO:4). It has been suggested that each of human NT pro-BNP, hBNP-32, and human proBNP-can circulate in human plasma (See, Tateyama et al., Biochem. Biophys. Res. Commun. 185 : 760-7 (1992); Hunt et al., Biochem. Biophys. Res. Commun. 214: 1175-83 (1995)).

CNP was first found in the brain, however, most of it originates in endothelial and renal cells. It is widely distributed in the vasculature, brain, bone and endothelium. Little if any CNP is present in the heart. Pro-CNP is a 103 amino acid peptide that is processed into either CNP-53 (amino acids 51 to 103) or CNP-22 (amino acids 82 to 103) that are the active peptides. Like ANP, CNP has a 17 amino acid ring closed by a disulfide bond between cysteine residues. In addition to this 17 amino acid ring structure, CNP-22 has an amino-terminal tail of 5 amino acids and contains no carboxy-terminal tail. CNP-53 is identical to CNP-22 except for a 31 amino acid extension at the amino terminal end.

As mentioned previously, DNP was isolated from the venom of the green mamba snake. The mature form of DNP is made up of 38 amino acids. DNP-like immunoreactivity (DNP-LI) has been reported in human plasma and the plasma concentration of DNP-LI has been found to be elevated in patients with congestive heart failure (See, Cataliotti, et al., Mayo Clin. Proc., 76:111-1119 (2001)). Additionally, it is also known that the infusion of synthetic DNP results in marked natriuresis and diuresis in association with increased plasma and urinary cyclic guanosine monophosphate. *Id.*

In humans, heart disease can stimulate the secretion of ANP and BNP. In fact, the secretion of ANP and BNP in humans typically reflects a change in cardiac function. Specifically, the secretion of ANP is typically accelerated when the atrium undergoes a load, while the biosynthesis and secretion of BNP is stimulated when the ventricle undergoes a load. Thereupon, both ANP and BNP are useful as indicators in the diagnosis of heart disease. However, despite this and over time, BNP has become recognized as a useful indicator in the diagnosis of heart disease, more so than ANP. For example, the blood concentration of BNP is only 1/6 of ANP in a normal subject but it becomes higher than ANP in patients of heart failure. Moreover, the blood concentration of BNP increases in the case of heart failure like ANP, and the plasma concentration of BNP often exceeds that of ANP, thus reflecting more accurately the severity of heart dysfunction. Moreover, BNP level in patients of heart failure sometimes increases to several tens times to several hundreds times of that of healthy normal subjects.

It is known that human proBNP, human NT-proBNP and hBNP can circulate and may be detected in test samples of patients suffering from cardiovascular disease, particularly heart failure. Both hBNP and human NT-proBNP are frequently used as markers to detect heart failure and to assess risk thereof in patients. However, the actual amount of each of the individual forms of BNP (i.e. human proBNP, human NT-proBNP and human BNP) that circulate is unclear due to the cross-reactivities of current commercial assays for these various forms (See, Liang F., et al., J. American College of Cardiology, 49(10):1071-1078 (2007)).

Additionally, it is known that human proBNP and human NT-proBNP can be glycosylated (See, Schellenberger, U. et al., Archives of Biochemistry and Biophysics, 451:160-166 (2006)), and these glycosylated forms have been isolated from human samples (See, Hammerer-Lercher A., et al., Clinical Chemistry, 54(5):858-865 (2008) and Seferian, K. et al., Clinical Chemistry, 54(5):866-873(2008)). There are seven sites of possible glycosylation confined to a 36-amino acid region within the N terminal portion of the peptide (from amino acid 36 through 71). Antibodies generated to this region may or may not bind to samples containing analyte human proBNP or NT-proBNP, depending on: 1) the immunogen used to raise the antibody; and 2) whether or not the analyte is glycosylated. Optional assays for human proBNP and NT-proBNP should use antibodies that avoid these regions.

The article of Lam et al. in Journal of American College of Cardiology, vol. 49, no. 11, 2007, pages 1193-1202 reports on the presence of alternate circulating proBNP and BNP forms as providing evidence for diverse proBNP and BNP processing in the general population and concludes that the physiologic consequences of these observations, both in terms of assay performance and endogenous BNP activity deserve further study.

In view thereof, there is a need in the art for new assays for quantifying the amount of human BNP, particularly assays having reduced cross-reactivity with other forms of the peptide, and especially as any clinical significance of variance in their individual circulating concentrations (e.g., vis-à-vis other forms) becomes understood. The present disclosure seeks to provide new assays and methods.

The methods can be used in qualitative or quantitative assays for human BNP, including assays carried out to assess the severity of cardiovascular disease, monitor progression of cardiovascular disease, or assess risk of progression of cardiovascular disease. These and other objects and advantages, as well as other additional features, will become apparent from the detailed description provided herein.

## SUMMARY

The present invention relates to an *in vitro* method of determining the severity of cardiovascular disease in a subject according to appended claim 1.

The present also invention relates to an *in vitro* method of monitoring the progression of cardiovascular disease in a subject according to appended claim 3.

The present invention further relates to an *in vitro* method of identifying a subject that would benefit from natriuretic peptide derivative treatment for cardiovascular disease according to appended claim 5.

In addition, the present invention relates to an *in vitro* method of determining if a subject has suffered a cardiovascular complication as a result of administration to said subject of one or more pharmaceutical compositions according to appended claim 7.

## DESCRIPTION OF THE FIGURES

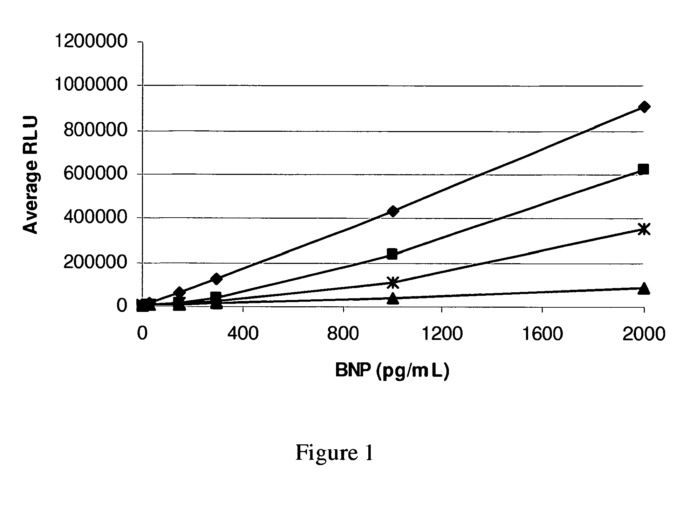

Figure 1 shows typical calibrations curves generated using hBNP peptide based calibrators and four selected BNP assays according to the immunoassays performed according to Example 1. In this Figure 1, -■- represents the 3-631-436 microparticle/106.3 AM1 conjugate; -▲- represents the 106.3 AM1 microparticle/8.1 conjugate; -♦- represents the 3-631-436 microparticle/8.1 conjugate; and ° represents the 106.3 AM1 microparticle/M1 conjugate.

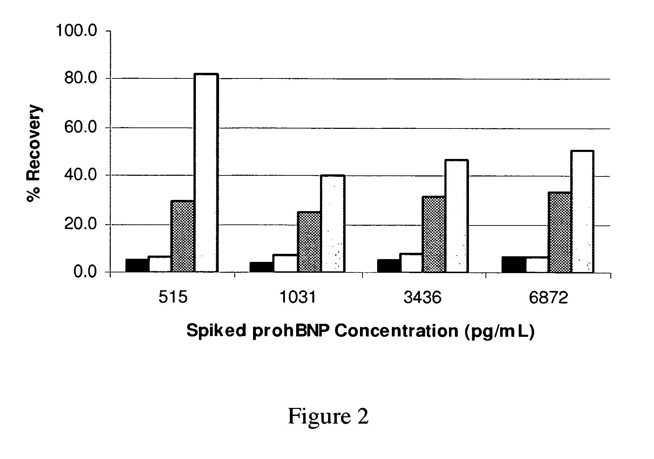

Figure 2 shows the mean % Recovery of spiked human proBNP peptide according to the immunoassays performed according to Example 1. In this Figure 2, the solid black box represents 3-631-436 microparticle/106.3 AM1 conjugate; the checkered box represents the 106.3AM1 microparticle/8.1 conjugate; the dotted box represents 3-631-436 microparticle/8.1 conjugate; and the hatched box represents the 106.3 AM1 microparticle/M1 conjugate.

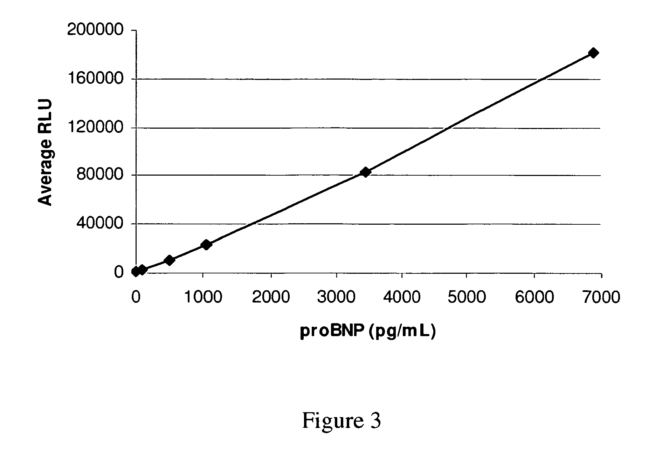

Figure 3 shows a typical calibration curve generated using human proBNP peptide based calibrators according to the immunoassay performed according to Example 2. In this Figure 3, -◇- represents the 106.3 AM1 microparticle/18 H5 conjugate.

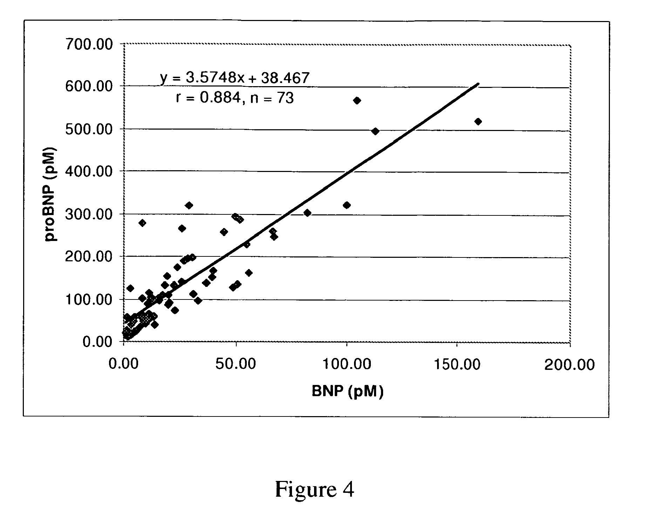

Figure 4 shows a plot of hBNP versus human proBNP concentrations as performed according to Example 4.

## DETAILED DESCRIPTION

The present disclosure relates to immunoassays for quantifying the amount of human B-type natriuretic peptide ("hBNP") present in a test sample being tested for or suspected of containing hBNP. Specifically, the immunoassays of the present disclosure exhibit reduced cross-reactivity with any human proB-type natriuretic peptide ("human proBNP"). The present disclosure relates to immunoassays for quantifying the amount of hBNP in a test sample wherein the immunoassays exhibit reduced cross-reactivity with any human proBNP or with any human N-terminal proB-type natriuretic peptide ("human NT-proBNP").

In an embodiment, the present invention relates to an in vitro method of determining the severity or progression of disease in a subject. In still yet another aspect, the present invention relates to an in vitro method of monitoring the progression of cardiovascular diseases in a subject. In another embodiment, the present invention relates to an in vitro method of identifying a subject that would benefit from natriuretic peptide derivative treatment for cardiovascular disease. In another embodiment, the present invention relates to an in vitro method of determining if a subject has suffered a cardiovascular complication as a result of administration to said subject of one or more pharmaceutical compositions.

In one embodiment according to the invention, provided is an in vitro method of determining the severity of cardiovascular disease in a subject, the in vitro method comprising the steps of:

(a) determining the amount of hBNP in a test sample according to an immunoassay for quantifying the amount of hBNP;

(b) determining the amount of human proBNP in said sample by any appropriate method (e.g., the method as described in Example 2);

(c) determining the molar ratio or weight ratio of the amount of human proBNP to the amount of hBNP in said sample; and

(d) correlating the molar ratio or weight ratio with severity of cardiovascular disease in the subject, wherein if the ratio is lower than a predetermined level the subject is determined to have increased severity of cardiovascular disease, and if the ratio is higher than a predetermined level the subject is determined to have reduced severity of cardiovascular disease;

wherein the immunoassay for determining the amount of hBNP has reduced cross-reactivity with any human pro B-type natriuretic peptide ("human proBNP") present in the test sample and comprises the steps of:

(I) contacting at least one capture antibody that binds to hBNP and that has been immobilized onto a solid phase to produce an immobilized antibody with said test sample to form a first mixture comprising an at least one capture antibody-hBNP complex, wherein said capture antibody comprises one or more antibodies having an equilibrium dissociation constant ($K_D$) of between about $3.0 \times 10^{-7}$ and about $1.0 \times 10^{-13}$ M;

(II) contacting said first mixture comprising the at least one capture antibody-hBNP complex with at least one detection antibody that binds to hBNP and that has been conjugated to a detectable label to form a second mixture comprising at least one

capture antibody-hBNP-at least one detection antibody complex, wherein the detection antibody comprises one or more antibodies having an equilibrium dissociation constant ($K_D$) of between about $3.0 \times 10^{-7}$ and about $1.0 \times 10^{-13}$ M; and

(III) determining the amount of the at least one capture antibody-hBNP-at least one detection antibody complex formed in step (II) by detecting the detectable label as a measure of the amount of hBNP contained in the test sample,

wherein the at least one capture antibody and the at least one detection antibody, when used together, exhibit a cross-reactivity of less than about 20% with any human proBNP present in the test sample.

In the above method, the cardiovascular disease can be coronary artery disease, peripheral vascular disease, hypertension, myocardial infarction or heart failure, among others.

In yet another embodiment according to the invention, provided is an in vitro method of monitoring the progression of cardiovascular disease in a subject, the in vitro method comprising the steps of:

(a) determining the amount of hBNP in the test according to an immunoassay for quantifying the amount of hBNP;

(b) determining the amount of human proBNP in said sample by any appropriate method (e.g., the method as described in Example 2);

(c) determining the molar ratio or weight ratio of the amount of human proBNP to the amount of hBNP in said sample; and

(d) correlating the molar ratio or weight ratio with progression of disease in the subject wherein the ratio is lower as compared to that in an earlier test sample from the subject with progression, and the ratio is unaltered or higher as compared to that in an earlier test sample from the subject with non-progression or improvement of cardiovascular disease;

wherein the immunoassay for determining the amount of hBNP has reduced cross-reactivity with any human pro B-type natriuretic peptide ("human proBNP") present in the test sample and comprises the steps of:

(I) contacting at least one capture antibody that binds to hBNP and that has been immobilized onto a solid phase to produce an immobilized antibody with said test sample to form a first mixture comprising an at least one capture antibody-hBNP complex, wherein said capture antibody comprises one or more antibodies having an equilibrium dissociation constant ($K_D$) of between about $3.0 \times 10^{-7}$ and about $1.0 \times 10^{-13}$ M;

(II) contacting said first mixture comprising the at least one capture antibody-hBNP complex with at least one detection antibody that binds to hBNP and that has been conjugated to a detectable label to form a second mixture comprising at least one capture antibody-hBNP-at least one detection antibody complex, wherein the detection antibody comprises one or more antibodies having an equilibrium dissociation constant ($K_D$) of between about $3.0 \times 10^{-7}$ and about $1.0 \times 10^{-13}$ M; and

(III) determining the amount of the at least one capture antibody-hBNP-at least one detection antibody complex formed in step (II) by detecting the detectable label as a measure of the amount of hBNP contained in the test sample,

wherein the at least one capture antibody and the at least one detection antibody, when used together, exhibit a cross-reactivity of less than about 20% with any human proBNP present in the test sample.

In the above method, the monitoring is done optionally following treatment for the cardiovascular disease, or prior to treatment (e.g., where the monitoring is done to assist with therapeutic decision such as when to initiate treatment).

An example of a subject that exhibits one or more clinical indicia associated with cardiovascular disease is a subject having a mutation in their corin or furin genes.

In still yet another aspect, the present invention relates to an in vitro method to identify a subject that would benefit from natriuretic peptide derivative treatment for cardiovascular disease, the in vitro method comprising the steps of:

(a) determining the amount of hBNP in a test sample from a subject that exhibits one or more clinical indicia associated with cardiovascular disease, which determination is carried out with an immunoassay for quantifying the amount of hBNP;

(b) determining the amount of human proBNP in said sample by any appropriate method (e.g., the method as described in Example 2);

(c) determining the molar ratio or weight ratio of the amount of human proBNP to the amount of hBNP in said sample;

(d) determining whether the molar ratio or weight ratio determined in step (c) is higher or lower than a predetermined level; and

(e) identifying whether the subject would benefit from natriuretic peptide derivative treatment based on the determination in step (d), wherein if the ratio is lower as compared to the predetermined level, the subject is identified as a subject that would not benefit from natriuretic peptide derivative treatment and further wherein, if the ratio is higher than a predetermined level, then the subject is identified as a subject that would benefit from natriuretic peptide derivative treatment;

wherein the immunoassay for determining the amount of hBNP has reduced cross-reactivity with any human pro B-type natriuretic peptide ("human proBNP") present in the test sample and comprises the steps of

(I) contacting at least one capture antibody that binds to hBNP and that has been immobilized onto a solid phase to produce an immobilized antibody with said test sample to form a first mixture comprising an at least one capture antibody-hBNP complex, wherein said capture antibody comprises one or more antibodies having an equilibrium dissociation constant ($K_D$) of between about $3.0 \times 10^{-7}$ and about $1.0 \times 10^{-13}$ M;

(II) contacting said first mixture comprising the at least one capture antibody-hBNP complex with at least one detection antibody that binds to hBNP and that has been conjugated to a detectable label to form a second mixture comprising at least one capture antibody-hBNP-at least one detection antibody complex, wherein the detection antibody comprises one or more antibodies having an equilibrium dissociation constant ($K_D$) of between about $3.0 \times 10^{-7}$ and about $1.0 \times 10^{-13}$ M; and

(III) determining the amount of the at least one capture antibody-hBNP-at least one detection antibody complex formed in step (II) by detecting the detectable label as a measure of the amount of hBNP contained in the test sample,

wherein the at least one capture antibody and the at least one detection antibody, when used together, exhibit a cross-reactivity of less than about 20% with any human proBNP present in the test sample.

An example of a human natriuretic peptide derivative that could be used to treat a subject is nesiritide.

In still yet another embodiment according to the invention, provided is an in vitro method of determining if a subject has suffered a cardiovascular complication as a

result of administration to said subject of one or more pharmaceutical compositions, the in vitro method comprising the steps of:

(a) determining the amount of human BNP in a test sample before the subject has been administered one or more pharmaceutical compositions with an immunoassay for quantifying the amount of hBNP present in a test sample;
(b) determining the amount of human proBNP in said sample by any appropriate method (e.g., the method as described in Example 2);
(c) determining the molar ratio or weight ratio of the amount of human proBNP to the amount of hBNP in said sample;
(d) determining the amount of human BNP in a second test sample from the subject after the subject has been administered one or more pharmaceutical compositions which determination is carried out with said immunoassay;
(e) determining the amount of human proBNP in said second test sample by any appropriate method (e.g., the method as described in Example 2);
(f) determining the molar ratio or weight ratio of the amount of human proBNP to the amount of hBNP in said second test sample; and
(g) comparing the molar ratio or weight ratio determined in step (c) with the molar or weight ratio in step (f), wherein if the molar ratio or weight ratio determined in step (c) is unchanged when compared to the molar ratio or weight ratio determined in step (f), then the subject is determined not to have suffered a cardiovascular complication as a result of the administration of one or more pharmaceutical compositions and further wherein if the molar ratio or weight ratio determined in step (c) is changed (either higher or lower) when compared to the molar ratio or weight ratio determined in step (f), then the subject is determined to have suffered a cardiovascular complication as a result of the administration of one or more pharmaceutical compositions;

wherein the immunoassay for determining the amount of hBNP has reduced cross-reactivity with any human pro B-type natriuretic peptide ("human proBNP") present in the test sample and comprises the steps of:

(I) contacting at least one capture antibody that binds to hBNP and that has been immobilized onto a solid phase to produce an immobilized antibody with said test sample to form a first mixture comprising an at least one capture antibody-hBNP complex, wherein said capture antibody comprises one or more antibodies having an equilibrium dissociation constant ($K_D$) of between about $3.0 \times 10^{-7}$ and about $1.0 \times 10^{-13}$ M;
(II) contacting said first mixture comprising the at least one capture antibody-hBNP complex with at least one detection antibody that binds to hBNP and that has been conjugated to a detectable label to form a second mixture comprising at least one capture antibody-hBNP-at least one detection antibody complex, wherein the detection antibody comprises one or more antibodies having an equilibrium dissociation constant ($K_D$) of between about $3.0 \times 10^{-7}$ and about $1.0 \times 10^{-13}$ M; and
(III) determining the amount of the at least one capture antibody-hBNP-at least one detection antibody complex formed in step (II) by detecting the detectable label as a measure of the amount of hBNP contained in the test sample,

wherein the at least one capture antibody and the at least one detection antibody, when used together, exhibit a cross-reactivity of less than about 20% with any human proBNP present in the test sample.

In a preferred embodiment, said immunoassay of the above methods further optionally comprises a wash step after step (a), step (b), or step (a) and step (b).

More specifically, in the above immunoassay, the at least one capture antibody and the at least one detection antibody, when used together, exhibit a cross-reactivity of less than about 15% with any human proBNP present in the test sample. Even more specifically, in the above immunoassay, the at least one capture antibody and the at least one detection antibody, when used together, exhibit a cross-reactivity of less than about 10% with any human proBNP present in the test sample.

Moreover, the capture antibody (e.g., the first capture antibody) and the detection antibody used in the immunoassays of the present disclosure can have an equilibrium dissociation constant of between about $2.5 \times 10^{-7}$ and about $5.0 \times 10^{-13}$ M, or between about $2.0 \times 10^{-7}$ and about $1.0 \times 10^{-12}$ M. The capture antibody (e.g., the first capture antibody) and the detection antibody can be a monoclonal antibody, a multispecific antibody, a human antibody, a fully humanized antibody, a partially humanized antibody, an animal antibody, a recombinant antibody, a chimeric antibody, a single-chain Fv, a single chain antibody, a single domain antibody, a Fab fragment, a F(ab')$_2$ fragment, a disulfide-linked Fv, an anti-idiotypic antibody, or a functionally active epitope-binding fragment thereof. Examples of antibodies that can be used as a first capture antibody include, but are not limited to, 106.3, BC203, M1, Clone 3 (3-631-436), AM1, AM5, AM8, 8.1 and 201.3. Examples of antibodies that can be used as a first detection antibody include, but are not limited to, 106.3, BC203, M1, Clone 3 (3-631-436), AM1, AM5, AM8, 8.1 and 201.3. Preferably, the capture antibody is Clone 3 (3-631-436). Preferably, the detection antibody is AM1 or 8.1.

## A. Definitions

As used herein, the singular forms "a," "an" and "the" include plural referents unless the context clearly dictates otherwise. For the recitation of numeric ranges herein, each intervening number there between with the same degree of precision is explicitly contemplated. For example, for the range 6-9, the numbers 7 and 8 are contemplated in addition to 6 and 9, and for the range 6.0-7.0, the numbers 6.0, 6.1, 6.2, 6.3, 6.4, 6.5, 6.6, 6.7, 6.8, 6.9 and 7.0 are explicitly contemplated.

a) Antibody

As used herein, the terms "antibody" and "antibodies" refer to monoclonal antibodies, multispecific antibodies, human antibodies, humanized antibodies (fully or partially humanized), animal antibodies (in one aspect, a bird (for example, a duck or goose), in another aspect, a shark or whale, in yet another aspect, a mammal, including a non-primate (for example, a cow, pig, camel, llama, horse, goat, rabbit, sheep, hamsters, guinea pig, cat, dog, rat, mouse, etc) and a non-human primate (for example, a monkey, such as a cynomologous monkey, a chimpanzee, etc)), recombinant antibodies, chimeric antibodies, single-chain Fvs (scFv), single chain antibodies, single domain antibodies, Fab fragments, F(ab') fragments, Fab" fragments, disulfide-linked Fvs (sdFv), and anti-idiotypic (anti-Id) antibodies (including, for example, anti-Id antibodies to antibodies of the present disclosure), and functionally active epitope-binding fragments of any of the above. In particular, antibodies include immunoglobulin molecules and immunologically active fragments of immunoglobulin molecules, namely, molecules that contain an antigen binding site. Immunoglobulin molecules can be of any type (for example, IgG, IgE, IgM, IgD, IgA and IgY), class (for example, $IgG_1$, $IgG_2$, $IgG_3$, $IgG_4$, $IgA_1$, and $IgA_2$) or subclass.

b) 8.1

As used herein, "8.1" refers to a monoclonal antibody or derivatives thereof produced by hybridoma cell line 8.1 which has been deposited with the American Type Culture Collection (A.T.C.C.) on February 21, 1996 and assigned A.T.C.C. Accession No. HB-12056. 8.1 and methods for making 8.1 are described in U.S. Patent No. 6,162,902. 8.1 binds to an epitope comprising amino acid residues 26-32 on hBNP.

c) 106.3

As used herein, "106.3" refers to a monoclonal antibody or derivatives thereof produced by hybridoma cell line 106.3 which has been deposited with the A.T.C.C. on February 14, 1996 and assigned A.T.C.C. Accession No. HB-12044. 106.3 and methods for making 106.3 are described in U.S. Patent No. 6,162,902. 106.3 binds to an epitope comprising amino acid residues 5-13 on hBNP. Two different equilibrium dissociation constants ($K_D$) have been reported for 106.3 namely, an equilibrium dissociation constant of about $0.32 \times 10^{-9}$ M and an equilibrium dissociation constant of about $1.0 \times 10^{-9}$ M.

d) 201.3

As used herein, "201.3" refers to a monoclonal antibody or derivatives thereof produced by hybridoma cell line 201.3 which has been deposited with the A.T.C.C. on February 14, 1996 and assigned A.T.C.C. Accession No. HB-12045. 201.3 and methods for making 201.3 are described in U.S. Patent No. 6,162,902. 201.3 binds to an epitope comprising amino acid residues 1-10 on hBNP.

e) AM1

As used herein, "AM1" or "106.3 AM1", "AM 1 106.3" or "106.3 L1 B24/H2 288" refers to a monoclonal antibody or derivatives thereof produced by hybridoma cell line 106.3 L1 B24/H2 288 which was deposited with the A.T.C.C. on September 20, 2005 and assigned A.T.C.C. Accession No. PTA-6987. AM1 and methods for making AM1 are described in U.S. Patent Application No. 1/595,625, filed on November 9, 2006. AM1 binds to an epitope comprising amino acid residues 5-13 on hBNP. Two different equilibrium dissociation constants ($K_D$) have been reported for AM1, namely, an equilibrium dissociation constant of about $0.14 \times 10^{-9}$ M and an equilibrium dissociation constant of about $1.9 \times 10^{-12}$ M.

f) AM5

As used herein, "AM5" refers to an antibody or derivatives thereof produced by Chinese Hamster Ovary (CHO) cell line AM5 (also known as BNP3-631-436AMSCHO893-214) which was deposited with the A.T.C.C. on April 24, 2007 and assigned A.T.C.C. Accession No. PTA-8369. AM5 and methods for making AM5 are described in U.S. Patent Application No. 11/745,963, filed on May 8, 2007. The equilibrium dissociation constant of AM5 is about $1.4 \times 10^{-10}$ M.

AM5 binds to an epitope comprising amino acid residues 13-18 on hBNP.

**g) AM8**

As used herein, "AM8" refers to an antibody or derivatives thereof produced by Chinese Hamster Ovary (CHO) cell line AM8 (also known as BNP3-631-436AM8CH0974-211) which was deposited with the A.T.C.C. on April 24, 2007 and assigned A.T.C.C. Accession No. PTA-8368. AM8 and methods for making AM8 are described in U.S. Patent Application No. 11/745,963, filed on May 8, 2007. The equilibrium dissociation constant of AM8 is about $1.0 \times 10^{-10}$ M. AM8 binds to an epitope comprising amino acid residues 13-18 on hBNP.

**h) BC203**

As used herein, "BC203" refers to a monoclonal antibody or derivatives thereof produced by hybridoma cell line BC203, which has been deposited with the Fermentation Research Institute at 1-3, Higashi 1-chome, Tsukuba-shi, Ibaraki-ken on August 20, 1991 and assigned Accession No. FERM BP-3515. BC203 and methods for making BC203 are described in U.S. Patent No. 6,677,124. BC203 binds to an epitope comprising amino acid residues 26-32 on hBNP. Two different equilibrium dissociation constants ($K_D$) have been reported for BC203, namely, an equilibrium dissociation constant of about $3.1 \times 10^{-8}$ M and an equilibrium dissociation constant of about $1.3 \times 10^{-9}$M.

**i) Clone 3 or BNP 3-631-436**

"Clone 3", "BNP 3-631-436 ms," "3-631-436" or "antibody 3-631-436" as used interchangeably herein, each refer to a monoclonal antibody or derivatives thereof produced by hybridoma cell line 3-631-436 which was deposited with the A.T.C.C. on December 21, 2004 and assigned A.T.C.C. Accession No. PTA-6476. Clone 3 and methods for making Clone 3 are described in U.S. Patent Publication 2006/0183154 published on August 17, 2006. Clone 3 binds to an epitope comprising amino acid residues 13-18 on hBNP. The equilibrium dissociation constant of Clone 3 is about $3.7 \times 10^{-10}$M.

**j) M1**

As used herein "M1" refers to a monoclonal antibody produced by hybridoma cell line M 1 which is commercially available from Strategic Diagnostics, Newark, Delaware (Catalog Number B910SMD01-D0). M1 binds to the carboxy (C) terminus on hBNP. The reported equilibrium dissociation constant of M1 is about $1.7 \times 10^{-7}$ M.

**k) Association Rate**

As used herein, the term "association rate", "$k_{on}$" or "$k_a$" as used interchangeably herein, refers to the value indicating the binding strength (degree) of an antibody to its target antigen or the rate of complex formation between mAb and antigen as shown by the below:

$$\text{Antibody (Ab)} + \text{Antigen (Ag)} \rightarrow \text{Ab-Ag}$$

Methods for determining association constants ($k_a$) are well known in the art. For example, a BIAcore® (biomolecular interaction analysis) assay can be used (e.g., instrument available from BIAcore International AB, a GE Healthcare company, Uppsala, Sweden). Additionally, a KinExA® (Kinetic Exclusion Assay) assay, available from Sapidyne Instruments (Boise, Idaho) can also be used.

**l) Cardiovascular Complication**

As used herein, the phrases "cardiovascular complication" or "suffering from a cardiovascular complication" as used interchangeably herein, refer to any cardiovascular disease or event. To the extent that a cardiovascular disease or event causes a secondary complication (e.g., pulmonary congestion or congested lung), such a secondary complication is also considered to be encompassed by the phrase "cardiovascular complication". A cardiovascular complication can be either "compensated" (e.g., compensated meaning that the regular oxygen need of the body of the subject can still be satisfied) or "decompensated" (e.g., decompensated meaning that the regular oxygen need of the body of the subject is not presently being satisfied). Also, phrases "cardiovascular complication" or "suffering from a cardiovascular complication" include deterioration of a pre-existing cardiovascular complication.

### m) Cardiovascular Disease

As used herein, the phrase "cardiovascular disease" refers to various clinical diseases, disorders or conditions involving the heart, blood vessels or circulation. The diseases, disorders or conditions may be due to atherosclerotic impairment of coronary, cerebral or peripheral arteries. Cardiovascular disease includes, but is not limited to, coronary artery disease, peripheral vascular disease, hypertension, myocardial infarction, heart failure, etc. For example, in heart failure, "increased severity" of cardiovascular disease refers to the worsening of disease as indicated by increased NYHA classification, to, for example, Class III or Class IV and "reduced severity" of cardiovascular disease refers to an improvement of the disease as indicated by reduced NYHA classification, from, for example, class III or IV to class II or I.

### n) Clinical Indicia

As used herein, the phrase "clinical indicia" refers to assays, test methods (such as imaging), standards (such as The New York Heart Association (NYHA) classification), biophysical measures (such as LDL concentration, HDL concentration, triglyceride concentration, blood pressure, body mass index, waist circumference, heart rate, fasting insulin concentration, fasting glucose concentration, diabetes status) and other biometric parameters (such as, but not limited to, race, gender, age, tobacco smoking status, previous history of cardiovascular disease, family history of cardiovascular disease, use of high blood pressure medication etc.) that provide an indicator of cardiovascular disease.

### o) Dissociation Rate

As used herein, the term "dissociation rate", "$k_{off}$" or "$k_d$" as used interchangeably herein, refers to the value indicating the dissociation strength (degree) of an antibody from its target antigen or separation of Ab-Ag complex over time into free mAb and antigen as shown by the below:

$$\text{Antibody (Ab)} + \text{Antigen (Ag)} \leftarrow \text{Ab-Ag}$$

Methods for determining dissociation constants ($k_d$) are well known in the art. For example, a Biacore® (Sweden) assay can be used. Additionally, a KinExA® (Kinetic Exclusion Assay) assay, available from Sapidyne Instruments (Boise, Idaho) can also be used.

### p) Equilibrium Dissociation Constant

As used herein, the term "equilibrium dissociation constant" or "$K_D$" as used interchangeably, herein, refers to the value obtained by dividing the dissociation rate ($k_{off}$) by the association rate ($k_{on}$). The association rate, the dissociation rate and the equilibrium dissociation constant are used to represent the binding affinity of an antibody to an antigen.

### q) Epitope

As used herein, the term "epitope" or "epitopes" refers to sites or fragments of a polypeptide or protein having antigenic or immunogenic activity in a subject. An epitope having immunogenic activity is a site or fragment of a polypeptide or protein that elicits an antibody response in an animal. An epitope having antigenic activity is a site or fragment of a polypeptide or protein to which an antibody immunospecifically binds as determined by any method well-known to those skilled in the art, for example by immunoassays.

### r) Heart Failure

As used herein, the phrase "heart failure" refers to a condition in which the heart cannot pump blood efficiently to the rest of the body. Heart failure may be due to damage to the heart or narrowing of the arteries due to infarction, cardiomyopathy (primary or secondary), hypertension, coronary artery disease, valve disease, birth defects or infection. Heart failure can further be described as chronic, congestive, acute, decompensated, systolic or diastolic. The New York Heart Association (NYHA) classification describes the severity of the disease based on functional capacity of the patient; NYHA class can progress and/or regress based on treatment or lack of response to treatment.

**s) Human Brain Natriuretic Peptide**

As used herein, the terms "human brain natriuretic peptide", "human BNP", "hBNP", "hBNP-32", "hBNP peptide", "hBNP polypeptide", or "B-type natriuretic peptide" used interchangeably herein, refer to a 32 amino acid molecule having the amino acid sequence shown in SEQ ID NO:4. The amino acid sequence shown in SEQ ID NO:4 is represented by amino acids 77-108 of the 108 amino acid sequence of human proBNP (SEQ ID NO:2).

**t) hBNP Fragment**

As used herein, the terms "hBNP fragment" "hBNP-32 fragment", "hBNP peptide fragment" or "human BNP fragment" as used interchangeably herein refers to a polypeptide that comprises at least six contiguous amino acids of SEQ ID NO:4. In one aspect, a hBNP fragment or hBNP peptide fragment refers to a peptide that comprises at least ten contiguous amino acids residues of SEQ ID NO:4; at least fifteen contiguous amino acids residues of amino acids of SEQ ID NO:4; at least 20 contiguous amino acids residues of SEQ ID NO:4; at least 25 contiguous amino acids residues of SEQ ID NO:4, or at least 30 contiguous amino acid residues of amino acids of SEQ ID NO:4. Examples of hBNP fragments or hBNP peptide fragments include, but are not limited to, amino acid sequences containing amino acids residues 1-31, 1-30, 1-29, 1-28, 1-27, 1-26, 1-25, 1-24, 1-23, 1-22, 1-21, 1-20, 1-19, 1-18, 1-17, 1-16, 1-15, 2-32, 2-31, 2-30, 2-29, 2-28, 2-27, 2-26, 2-25, 2-24, 2-23, 2-22, 2-21, 2-20, 2-19, 2-18, 2-17, 2-16, 2-15, 2-14, 2-13, 2-12, 2-11, 2-10, 2-9, 2-8, 2-7, 3-32, 3-31, 3-30, 3-29, 3-28, 3-27, 3-26, 3-25, 3-24, 3-23, 3-32, 3-21, 3-20, 3-19, 3-18, 3-17, 3-16, 3-15, 3-14, 3-13, 3-12, 3-11, 3-10, 3-9, 3-8, 4-32, 4-31, 4-30, 4-29, 4-28, 4-27, 4-26, 4-25, 4-24, 4-23, 4-22, 4-21, 4-20, 4-19, 4-18, 4-17, 4-16, 4-15, 4-14, 4-13, 4-12, 4-11, 4-10, 4-9, 5-32, 5-31, 5-30, 5-29, 5-28, 5-27, 5-26, 5-25, 5-24, 5-23, 5-22, 5-21, 5-20, 5-19, 5-18, 5-17, 5-16, 5-15, 5-14, 5-13, 5-12, 5-11, 5-10, 6-32, 6-31, 6-30, 6-29, 6-28, 6-27, 6-26, 6-25, 6-24, 6-23, 6-22, 6-21, 6-20, 6-19, 6-18, 6-17, 6-16, 6-15, 6-14, 6-13, 6-12, 6-11, 7-32, 7-31, 7-30, 7-29, 7-28, 7-27, 7-26, 7-25, 7-24, 7-23, 7-22, 7-21, 7-20, 7-19, 7-18, 7-17, 7-16, 7-15, 7-14, 7-13, 7-12, 8-32, 8-31, 8-30, 8-29, 8-28, 8-27, 8-26, 8-25, 8-24, 8-23, 8-22, 8-21, 8-20, 8-19, 8-18, 8-17, 8-16, 8-15, 8-14, 8-13, 9-32, 9-31, 9-30, 9-29, 9-28, 9-27, 9-26, 9-25, 9-24, 9-23, 9-22, 9-21, 9-20, 9-19, 9-18, 9-17, 9-16, 9-15, 9-14, 10-32, 10-31, 10-30, 10-29, 10-28, 10-27, 10-26, 10-25, 10-24, 10-23, 10-22, 10-21, 10-20, 10-19, 10-18, 10-17, 10-16, 10-15, 11-32, 11-31, 11-30, 11-29, 11-28, 11-27, 11-26, 11-25, 11-24, 11-23, 11-22, 11-21, 11-20, 11-19, 11-18, 11-17 or 11-16 of SEQ ID NO:4.

**u) Human Natriuretic Peptide Analog**

As used herein, the phrase "human natriuretic peptide analog" refers to a biologically active analog of a human natriuretic peptide (e.g., human BNP). For example, a biologically active human natriuretic peptide analog can be a human natriuretic peptide with truncations, deletions, insertions, substitutions, replacements, side chain extensions, and fusion proteins, or combinations of the foregoing which do not eliminate the biological activity of the original compound. Human natriuretic peptide analogs can be obtained by various means. For example, certain amino acids can be substituted for other amino acids in the native natriuretic peptide structure without eliminating interactive binding capacity. Examples of human natriuretic peptide analogs and methods for making such analogs are described in U.S. Patent Application No. 2006/0172933.

**v) Human Natriuretic Peptide Conjugate**

As used herein, the phrase "human natriuretic peptide conjugate" refers to human natriuretic peptide or human natriuretic peptide fragment that includes at least one modifying moiety or at least one reactive entity attached thereto. Modifying moieties are moieties that modify a human natriuretic peptide or a human natriuretic peptide fragment (e.g., hBNP or hBNP fragment). Example of modifying moieties include, but are not limited to, moieties that effect stability, solubility, and/or biological activity (e.g., hydrophilic polymers or oligomers, amphiphilic polymers or oligomers, and lipophilic polymers or oligomers), hydrophilic moieties, polyethylene glycol moieties, biocompatible water soluble moieties, polycationic moieties, amphiphilic moieties, polyethylene glycol/alkyl modifying moieties, etc. (each of which are described in U.S. Patent Application No. 2006/0172933).

Human natriuretic peptides or human natriuretic peptide fragments can be chemically modified (by covalent bonding) by coupling to a reactive entity as described in U.S. Patent Application No. 2004/0266673. The reactive entity is capable of forming a covalent bond with a blood component, preferably a blood protein. The covalent bond is generally formed between the reactive entity and an amino group, a hydroxyl group, or a thiol group on the blood component. The amino group can form a covalent bond with reactive entities like carboxy, phosphoryl or acyl; the hydroxyl group preferably forms a covalent bond with reactive entities like activated esters; and the thiol group preferably forms a covalent bond with reactive entities like esters or mixed anhydrides. The preferred blood components are mobile blood components like serum albumin, immunoglobulins, or combinations thereof, and the preferred reactive entity comprises anhydrides like maleimide or maleimido-containing groups.

Methods for conjugating a modifying moiety to a base molecules, such as a human natriuretic peptides (e.g., human BNP) are well known in the art. For example, strategies for conjugating a modifying moiety to human natriuretic peptide are disclosed in U.S. Patent Application No. 2006/0172933. Methods for chemically modifying human natriuretic peptides to a reactive entity are described in U.S. Patent Application No. 2004/0266673.

### w) Human Natriuretic Peptide Derivative

As used herein, the phrase "human natriuretic derivative" refers to a human natriuretic peptide analog, a human natriuretic peptide conjugate or a recombinant form of a human natriuretic peptide (e.g., a recombinant form of human BNP (SEQ ID NO:4) (e.g, nesiritide)).

### x) Immunodiagnostic Reagent

As used herein, the term "immunodiagnostic reagent" refers to one or more antibodies that specifically bind to a region of hBNP.

### y) Pharmaceutical Composition

As used herein, the term "pharmaceutical composition" refers to any agent or drug, whether a small molecule (e.g., a drug containing an active agent) or biologic that can be used to treat a subject suffering from a disease or condition that requires treatment. Examples of pharmaceutical compositions, include, but are not limited to, antineoplastics (chemotherapeutics), antidepressants (e.g., tricyclic antidepressants), multiple sclerosis drugs, anesthetics, interferons, hormones, HIV-antiviral drugs, etc. as well as any combinations thereof. Generally, a pharmaceutical composition may be one that is known or suspected of impacting the cardiovascular system, or one that unexpectedly or surprisingly impacts the cardiovascular system.

### z) Pre-Pro Peptide Precursor of Human BNP

As used herein, the term "pre-pro peptide precursor of human BNP" or "human pre-proBNP" refers to a 134 amino acid molecule having the amino acid sequence shown in SEQ ID NO:1.

### aa) Pro Peptide of Human BNP

As used herein, the term "pro peptide of human BNP" or "human proBNP" refers to a 108 amino acid molecule having the amino acid sequence shown in SEQ ID NO:2. Human proBNP is derived from human pre-proBNP.

### bb) N-Terminal Pro Peptide of Human BNP

As used herein, the term "N-terminal pro peptide of human BNP," "human NT-pro B-type natriuretic peptide" or "human NT-proBNP" refers to a 76 amino acid molecule having the amino acid sequence shown in SEQ ID NO:3. Human NT-proBNP is derived from human proBNP (SEQ ID NO:2).

### cc) Subject

As used herein, the terms "subject" and "patient" are used interchangeably, although a subject of the disclosure herein need not necessarily be undergoing or have undergone medical treatment at the time of the immunoassay. As used herein, the terms "subject" and "subjects" refer to an animal, in one aspect, a bird (for example, a duck or goose), in another aspect, a shark or whale, or in a further aspect, a mammal including, a non-primate (for example, a cow, pig, camel, llama, horse, goat, rabbit, sheep, hamsters, guinea pig, cat, dog, rat, and mouse) and a primate (for example, a monkey, such as a cynomologous monkey, chimpanzee, and a human). Preferably, the subject is a human.

### dd) Test Sample

As used herein, the term "test sample" refers to a biological sample derived from tissues, serum, plasma, whole blood, lymph, CNS fluid, urine or other bodily fluids of a subject that is being tested for, and/or may be suspected of containing hBNP. The test sample can be prepared using routine techniques known to those skilled in the art.

**ee) Readback**

The term "readback" refers to the concentration of a given analyte (e.g., proBNP) read off a calibration curve (e.g., a BNP calibration curve). Optimally, when the analyte is not identical to the calibrator (e.g., analyte proBNP and calibrator BNP) the readback is corrected for the difference in molecular weight.

**ff) Reduced Cross-Reactiity**

The term "reduced cross-reactivity" as used herein refers to an immunoassay for human BNP in a test sample that employs one or more antibodies that when used together exhibit a reduction in any binding to proBNP or NT-proBNP contained in the test sample, as compared with any other (e.g., at least one, optionally at least two) human BNP assay and/or human BNP spiking study as known by one skilled in the art. The reduction can range from about 0.1 % to about 20%, optionally from about 1 % to about 20%, about 0.1% to about 15%, about 1% to about 15%, about 0.1% to about 10% or about 1% to about 10%.

**gg) Predetermined Level**

As used herein, the term "predetermined level" refers generally at an assay cutoff value that is used to assess diagnostic results by comparing the assay results against the predetermined level, and where the predetermined level already that has been linked or associated with various clinical parameters (e.g., severity of disease, progression/ nonprogression/improvement, etc.). The present disclosure provides exemplary predetermined levels, and describes the initial linkage or association of such levels with clinical parameters for exemplary immunoassays as described herein. However, it is well known that cutoff values may vary dependent on the nature of the immunoassay (e.g., antibodies employed, etc.). It further is well within the ordinary skill of one in the art to adapt the disclosure herein for other immunoassays to obtain immunoassay-specific cutoff values for those other immunoassays based on this description. Whereas the precise value of the predetermined level (cutoff) may vary between assays, the correlations as described herein should be generally applicable.

**B. Immunoassays**

As mentioned briefly herein, the present disclosure relates to immunoassays that can be used for the qualitative detection and/or quantification of hBNP or hBNP fragment in a test sample. The immunoassays described herein exhibit reduced cross-reactivity with any human proBNP or human NT-proBNP that may be contained in the test sample.
The immunoassays of the present disclosure can be conducted using any format known in the art, such as, but not limited to, a sandwich format.
In certain embodiments of the present disclosure, at least two antibodies are employed to separate and quantify hBNP or hBNP fragment in a test sample. More specifically, the at least two antibodies bind to certain epitopes of hBNP or hBNP fragment forming an immune complex which is referred to as a "sandwich". Generally, in the immunoassays one or more antibodies can be used to capture the hBNP or hBNP fragment in the test sample (these antibodies are frequently referred to as a "capture" antibody or "capture" antibodies) and one or more antibodies can be used to bind a detectable (namely, quantifiable) label to the sandwich (these antibodies are frequently referred to as the "detection antibody," "detection antibodies," a "conjugate" or "conjugates").
The inventors have discovered that excellent immunoassays, particularly, sandwich assays, can be performed using certain combinations of antibodies as the capture and detection antibodies. More specifically, the antibodies used in particular embodiments of the present disclosure as capture and detection antibodies each have an equilibrium dissociation constant ($K_D$) ranging from between about $3.0 \times 10^{-7}$ and about $1.0 \times 10^{-13}$ M, between about $2.5 \times 10^{-7}$ and about $5.0 \times 10^{-13}$ M, or between about $2.0 \times 10^{-7}$ and about $1.0 \times 10^{-12}$ M. Examples of antibodies that have an equilibrium dissociation constant ($K_D$) within the above-described ranges and that can be used as capture and detection antibodies in the immunoassays of the present disclosure include, but are not limited to, 106.3, BC203, Clone 3, M1, AM1, AM5, 201.3, AM8 or 8.1.
Certain capture antibody and conjugate antibody combinations are not preferred for use together in a sandwich assay as described herein because they share the same or overlapping epitopes. Groups of antibodies with the same or overlapping epitopes are shown below in Table A:

Table A

| Group | Antibodies |
|-------|-----------|
| A | Clone 3, AM5 or AM8 |
| B | 106.3, AM 1 or 201.3 |
| C | BC203, M1 or 8.1 |

Generally, antibodies in different groups (e.g., arbitrarily designated "A", "B" or "C") will pair with each other. For example, generally, an antibody from Group A will pair with an antibody from Group B. In some cases and assay formats, however, it is possible to use for sandwich formation two antibodies that have overlapping or contiguous epitopes. In other words, Table A is exemplary only, not intended as limiting in terms of antibody pairings.

Immunoassays performed as described herein that employ as at least one capture antibody and at least one detection antibody each having an equilibrium dissociation constant ($K_D$) ranging from between about $3.0 \times 10^{-7}$ and about $1.0 \times 10^{-13}$ M, between about $2.5 \times 10^{-7}$ and about $5.0 \times 10^{-13}$ M, or between about $2.0 \times 10^{-7}$ and about $1.0 \times 10^{-12}$ M and, when said at least one capture antibody and at least one detection antibody are used together, exhibit reduced cross-reactivity with any human proBNP that may be present in the test sample. The immunoassay exhibits a cross-reactivity of less than about 20% with any human proBNP that may be present in the test sample. More preferably, the immunoassay exhibits a cross-reactivity of less than about 19%, less than about 18%, less than about 17%, less than about 16%, less than about 15%, less than about 14%, less than about 13%, less than about 12%, less than about 11%, less than about 10%, less than about 9%, less than about 8%, less than about 7%, less than about 6%, less than about 5%, less than about 4%, less than about 3%, less than about 2%, or less than about 1% (e.g., such as about 0.1%) with any human proBNP that may be present in the test sample.

In another embodiment of the disclosure, immunoassays performed as described herein that employ as at least one capture antibody and at least one detection antibody each having an equilibrium dissociation constant ($K_D$) ranging from between about $3.0 \times 10^{-7}$ and about $1.0 \times 10^{-13}$ M, between about $2.5 \times 10^{-7}$ and about $5.0 \times 10^{-13}$ M, or between about $2.0 \times 10^{-7}$ and about $1.0 \times 10^{-12}$ M exhibit a reduced cross-reactivity with an human proBNP or human NT-proBNP that may be present in the test sample. Preferably, the immunoassay exhibits a cross-reactivity of less than about 20% with any human proBNP or human NT-proBNP that may be present in the test sample.

The immunoassay exhibits a cross-reactivity of less than about 19%, less than about 18%, less than about 17%, less than about 16%, less than about 15%, less than about 14%, less than about 13%, less than about 12%, less than about 11%, less than about 10%, less than about 9%, less than about 8%, less than about 7%, less than about 6%, less than about 5%, less than about 4%, less than about 3%, less than about 2%, or less than about 1% with any human proBNP or human NT-proBNP that may be present in the test sample.

The test sample being tested for (e.g., suspected of containing) hBNP or a hBNP fragment can be contacted with at least one capture antibody (or antibodies) and at least one detection antibody (or antibodies) either simultaneously or sequentially and in any order. For example, the test sample can be first contacted with at least one capture antibody and then (sequentially) with at least one detection antibody. Alternatively, the test sample can be first contacted with at least one detection antibody and then (sequentially) with at least one capture antibody. In yet another alternative, the test sample can be contacted simultaneously with a capture antibody and a detection antibody.

In the sandwich assay format, a test sample suspected of containing hBNP or hBNP fragment is first brought into contact with the at least one first capture antibody having an equilibrium dissociation constant ranging from between about $3.0 \times 10^{-7}$ and about $1.0 \times 10^{-13}$ M, between about $2.5 \times 10^{-7}$ and about $5.0 \times 10^{-13}$ M, or between about $2.0 \times 10^{-7}$ and about $1.0 \times 10^{-12}$ M under conditions which allow the formation of a first antibody-hBNP complex. If more than one capture antibody is used, a first multiple capture antibody-hBNP complex is formed. In a sandwich assay, the antibodies, preferably, the at least one capture antibody, are used in molar excess amounts of the maximum amount of hBNP or hBNP fragment expected in the test sample. For example, from about 5 μg/mL to about 1 mg/mL of antibody per mL of buffer (e.g., microparticle coating buffer) can be used.

Prior; to contacting the test'sample with the at least one capture antibody (e.g., the first capture antibody), the at least one capture antibody can be bound to a solid support which facilitates the separation of the first antibody-hBNP complex from the test sample. Any solid support known in the art can be used, including but not limited to, solid supports made out of polymeric materials in the forms of wells, tubes or beads. The antibody (or antibodies) can be bound to the solid support by adsorption, by covalent bonding using a chemical coupling agent or by other means known in the art, provided that such binding does not interfere with the ability of the antibody to bind hBNP or hBNP fragment. Alternatively, the antibody (or antibodies) can be bound with microparticles that have previously been coated with streptavidin (for example, using Power-Bind™-SA-MP streptavidin coated microparticles, available from Seradyn, Indianapolis, Indiana). Alternatively, the antibody (or antibodies) can be bound using microparticles that have been previously coated with anti-species specific monoclonal antibodies. Moreover, if necessary, the solid support can be derivatized to allow reactivity with various functional groups on the antibody. Such derivatization requires the use of certain coupling agents such as, but not limited to, maleic anhydride, N-hydroxysuccinimide and 1-ethyl-3-(3-dimethylaminopropyl)carbodiimide.

After the test sample being tested for and/or suspected of containing hBNP or an hBNP fragment is brought into contact with the at least one capture antibody (e.g., the first capture antibody), the mixture is incubated in order to allow for the formation of a first antibody (or multiple antibody)-hBNP complex. The incubation can be carried out at a pH of from about 4.5 to about 10.0, at a temperature of from about 2°C to about 45°C, and for a period from at least about one (1) minute to about eighteen (18) hours, preferably from about 1 to 20 minutes, most preferably from about 2-4 minutes. The immunoassay described herein can be conducted in one step (meaning the test sample, at least one capture antibody and at least one detection antibody are all added sequentially or simultaneously to a reaction vessel) or in more than one step, such as two steps, three steps, etc.

After formation of the (first/multiple) capture antibody-hBNP complex, the complex is then contacted with at least one detection antibody (under conditions which allow for the formation of a (first/multiple) capture antibody-hBNP-second antibody detection complex). The at least one detection antibody can be the second, third, fourth, etc. antibodies used in the immunoassay. If the capture antibody-hBNP complex is contacted with more than one detection antibody, then a (first/multiple) capture antibody-hBNP-(multiple) detection antibody complex is formed. As with the capture antibody (e.g., the first capture antibody), when the at least second (and subsequent) detection antibody is brought into contact with the capture antibody-hBNP complex, a period of incubation under conditions similar to those described above is required for the formation of the (first/multiple) capture antibody-hBNP-(second/multiple) detection antibody complex. Preferably, at least one detection antibody contains a detectable label. The detectable label can be bound to the at least one detection antibody (e.g., the second detection antibody) prior to, simultaneously with or after the formation of the (first/multiple) capture antibody-hBNP-(second/multiple) detection antibody complex. Any detectable label known in the art can be used. For example, the detectable label can be a radioactive label, such as, $^3$H, $^{125}$I, $^{35}$S, $^{14}$C, $^{32}$P, $^{33}$P, an enzymatic label, such as horseradish peroxidase, alkaline phosphatase, glucose 6-phosphate dehydrogenase, etc., a chemiluminescent label, such as, acridinium esters, luminol, isoluminol, thioesters, sulfonamides, phenanthridinium esters, etc. a fluorescence label, such as, fluorescein (5-fluorescein, 6-carboxyfluorescein, 3'6-carboxyfluorescein, 5 (6)-carboxyfluorescein, 6-hexachlorofluorescein, 6-tetrachlorofluorescein, fluorescein isothiocyanate, etc.), rhodamine, phycobiliproteins, R-phycoerythrin, quantum dots (zinc sulfide-capped cadmium selenide), a thermometric label or an immuno-polymerase chain reaction label. An introduction to labels, labeling procedures and detection of labels is found in Polak and Van Noorden, Introduction to Immunocytochemistry, 2nd ed., Springer Verlag, N.Y. (1997) and in Haugland, Handbook of Fluorescent Probes and Research Chemicals (1996), which is a combined handbook and catalogue published by Molecular Probes, Inc., Eugene, Oregon. In addition, more than one label can be used. For example, double conjugates can be used, each of which contain different labels. For example, one conjugate antibody can contain biotin and the second conjugate can be an anti-biotin antibody labeled with acridinium. Other variations would be easily recognized by one of ordinary skill in the art.

The detectable label can be bound to the antibodies either directly or through a coupling agent. An example of a coupling agent that can be used is EDAC (1-ethyl-3-(3-dimethylaminopropyl) carbodiimide, hydrochloride) that is commercially available from Sigma-Aldrich, St. Louis, MO. Other coupling agents that can be used are known in the art. Methods for binding a detectable label to an antibody are known in the art. Additionally, many detectable labels can be purchased or synthesized that already contain end groups that facilitate the coupling of the detectable label to the antibody, such as, N10-(3-sulfopropyl)-N-(3-carboxypropyl)-acridinium-9-carboxamide active esters, otherwise known as CPSP-Acridinium Ester or N 10-(3-sulfopropyl)-N-(3-sulfopropyl)-acridinium-9-carboxamide active ester, otherwise known as SPSP-Acridinium Ester.

The (first/multiple)capture antibody-hBNP-(second/multiple) detection antibody complex can be, but does not have to be, separated from the remainder of the test sample prior to quantification of the label. For example, if the at least one capture antibody (e.g., the first capture antibody) is bound to a solid support, such as a well or a bead, separation can be accomplished by removing the fluid (of the test sample) from contact with the solid support. Alternatively, if the at least first capture antibody is bound to a solid support it can be simultaneously contacted with the hBNP-containing sample and the at least one second detection antibody to form a first (multiple) antibody-hBNP-second (multiple) antibody complex, followed by removal of the fluid (test sample) from contact with the solid support. If the at least one first capture antibody is not bound to a solid support, then the (first /multiple) capture antibody-hBNP-(second/multiple) detection antibody complex does not have to be removed from the test sample for quantification of the amount of the label.

After formation of the labeled capture antibody-hBNP-detection antibody complex (e.g., the first capture antibody-hBNP-second detection antibody complex), the amount of label in the complex is quantified using techniques known in the art. For example, if an enzymatic label is used, the labeled complex is reacted with a substrate for the label that gives a quantifiable reaction such as the development of color. If the label is a radioactive label, the label is quantified using a scintillation counter. If the label is a fluorescent label, the label is quantified by stimulating the label with a light of one color (which is known as the "excitation wavelength") and detecting another color (which is known as the "emission wavelength") that is emitted by the label in response to the stimulation. If the label is a chemiluminescent label, the label is quantified detecting the light emitted either visually or by using luminometers, x-ray film, high speed photographic film, a CCD camera, etc. Once the amount of the label in the complex has been quantified, the concentration of hBNP or hBNP fragment in the test sample is determined by use of a standard curve that has been generated using serial dilutions of hBNP or hBNP fragment of known concentration. Other than using serial dilutions of hBNP or hBNP fragment the standard curve can be generated gravimetrically, by mass spectroscopy and by other techniques known in the art.

## C. Methods for Determining Certain Ratios in a Test Sample

The present disclosure pertains to methods for determining the molar ratio or weight ratio of (a) the amount of hBNP to the amount of human proBNP in a test sample or (b) the amount of human proBNP to hBNP in a test sample. Specifically, the methods of the present disclosure involve determining the amount of hBNP in a test sample. Any appropriate methods for determining or quantifying the amount of hBNP in a test sample can be used. Such methods are well known in the art. For example, such a method can be an immunoassay, such as the immunoassay described herein (e.g., in Section B, above). Additionally, the methods of the present disclosure also involve determining or quantifying the amount of human proBNP in a test sample. Any appropriate methods for determining or quantifying the amount of human proBNP in a test sample can be used. Such methods are well known in the art. For example, such a method can began immunoassay, such as the immunoassay described herein (e.g., in Section B, above). Alternatively, a method such as that described in Example 2 can be used. The order in which the amount of hBNP or human proBNP in a test sample are determined or quantified is not critical. The molar ratio or weight ratio that can be determined can be (i) the amount of hBNP to the amount of human proBNP in said sample; or (ii) the amount of human proBNP to the amount of hBNP in said sample. Preferably the molar ratio or weight ratio that is determined is the amount of human proBNP. to the amount of hBNP in a test sample.

The molar ratio of human proBNP/hBNP determined pursuant to the above-described method can range from about 1.0 to about 50.0. Preferably, the range is from about 1.20 to about 45.0. The weight ratio of human proBNP/hBNP determined pursuant to the above-described method can range from about 2.0 to about 150.0. Preferably, the range is from 4.0 about to about 145.0. The molar ratio of hBNP/human proBNP determined pursuant to the above-described method can range from about 0.02 to about 0.72. The weight ratio of hBNP/human proBNP determined pursuant to the above-described method can range from about 0.007 to about 0.21.

The molar ratio or weight ratio determined using the methods described herein can be useful to provide an indicator of the clinical status (i.e., severity or progression of disease) of a subject. For example, the molar ratio or weight ratio determined using the methods of the present disclosure can be used to determine whether or not subject is suffering from a disease such as heart failure. Alternatively, the molar ratio or weight ratio described herein can be used to determine whether a subject suffering from heart failure should be classified in any of New York Heart Association (NYHA) Classifications I, II, III or IV or whether a subject classified as certain New York Heart Association Classification has progressed to a different New York Heart Association Classification (e.g., the subject was initially classified as New York Heart Association Classification II and then the subject progress to New York Heart Association Classification III). Specifically, the severity or progression of disease, such as cardiovascular disease, in a subject can be determined using a method comprising the steps of:

(a) providing a test sample from a subject;
(b) determining the amount of hBNP in the test sample according to any of the immunoassays described herein (e.g., previously, in Section B);
(c) determining the amount of human proBNP in said sample;
(d) determining the molar ratio or weight ratio of the amount of human proBNP to the amount of hBNP in said sample; and
(e) correlating the molar ratio or weight ratio with severity or progression of disease in the subject. Specifically, when correlating the molar ratio or weight ratio with severity of cardiovascular disease in a subject, the ratio is lower than a predetermined level with increased severity and the ratio is higher than a predetermined level with reduced severity . When correlating the molar ratio or weight ratio with progression of disease in a subject, the ratio is lower as compared to that in an earlier test sample from the subject with progression, and the ratio is unaltered or higher as compared to that in an earlier test sample from the subject with non-progression or improvement of cardiovascular disease. The progression of disease, such as cardiovascular disease, can be monitored either before treatment is commenced in a subject or after commencement of treatment in a subject.

Additionally, the molar ratio or weight ratio determined using the methods described herein can be used to determine or identify whether a subject might have a mutation in their corin or furin genes. Corin and furin are serine proteases that are known to cleave the proforms of naturietic peptides (e.g, corin cleaves both proANP and proBNP and furin cleaves proCNP and proBNP) into their active forms (ANP, BNP, CNP) and their N-terminal inactive peptides. Mutations to these genes can result in incomplete processing of the prohormones as they are needed leading to lower circulating amounts of active peptide. Therefore, a subject having a corin or furin mutation would be expected to have a higher human proBNP to hBNP ratio then a subject lacking the mutation or having a wild-type allele. This could help identify subjects that could benefit from treatment for cardiovascular disease, especially to determine or identify a subject that would benefit from natriuretic peptide derivative treatment for cardiovascular disease. For example, such a method can comprise the steps of:

(a) obtaining a test sample from the subject that exhibits one or more clinical indicia associated with cardiovascular disease (such as, for example, a subject having a mutation in their corin or furin genes);
(b) determining the amount of human BNP in the test sample according to any of the immunoassays described herein (e.g., previously, in Section B);
(c) determining the amount of human proBNP in said sample;
(d) determining the molar ratio or weight ratio of the amount of human proBNP to the amount of hBNP in said sample;
(e) determining whether the molar ratio or weight ratio determined in step (d) is higher or lower than a predetermined level; and
(f) identifying whether the subject would benefit from natriuretic peptide derivative treatment based on the determination in step (e). Specifically, if the ratio determined in step (d) is lower as compared to the predetermined level, the subject would be identified as a subject that would not benefit from natriuretic peptide derivative treatment. However, if the ratio determined in step (d) is higher than the predetermined level, then the subject would be identified as a subject that would benefit from natriuretic peptide derivative treatment. An example of a human natriuretic peptide derivative that could be used to treat a subject is nesiritide. The type and amount of treatment for such a subject could readily be determined by one skilled in the art using routine techniques.

Moreover, the molar ratio or weight ratio determined using the methods described herein can be used to determine if a subject has suffered a cardiovascular complication as a result of administration to said subject of one or more pharmaceutical compositions. For example, such a method can comprise the steps of:

(a) obtaining a first test sample from the subject before the subject has been administered one or more pharmaceutical compositions;
(b) determining the amount of human BNP in the test sample according to any of the immunoassays described herein (e.g., previously, in Section B);
(c) determining the amount of human proBNP in said sample;
(d) determining the molar ratio or weight ratio of the amount of human proBNP to the amount of hBNP in said sample;
(e) obtaining a second test sample from the subject after the subject has been administered one or more pharmaceutical compositions;
(f) determining the amount of human BNP in the second test sample according to any of the immunoassays described herein (e.g., previously, in Section B);
(g) determining the amount of human proBNP in said second test sample;
(h) determining the molar ratio or weight ratio of the amount of human proBNP to the amount of hBNP in said second test sample; and
(i) comparing the molar ratio or weight ratio determined in step (d) with the molar or weight ratio in step (h). Specifically, if the molar ratio or weight ratio determined in step (d) is unchanged when compared to the molar ratio or weight ratio determined in step (h), then the subject is determined not to have suffered a cardiovascular complication as a result of the administration of one or more pharmaceutical compositions. If the molar ratio or weight ratio determined in step (d) is changed (either higher or lower) when compared to the molar ratio or weight ratio determined in step (h), then the subject is determined to have suffered a cardiovascular complication as a result of the administration of one or more pharmaceutical compositions.

## D. Adaptations of the Methods

The disclosure herein also can be adapted for use in a variety of automated and semi-automated systems (including those wherein the solid phase comprises a microparticle), as described, e.g., in U.S. Patent Nos. 5,089,424 and 5,006,309, and as, e.g., commercially marketed by Abbott Laboratories (Abbott Park, IL) including but not limited to Abbott's ARCHITECT®, AxSYM®, IMx®, PRISM®, and Quantum™ II instruments, as well as other platforms. Moreover, the disclosure optionally is adaptable for the Abbott Laboratories commercial Point of Care (i-STAT™) electrochemical immunoassay system for performing sandwich immunoassays. Immunosensors, and their methods of manufacture and operation in single-use test devices are described, for example in, U.S. Patent No. 5,063,081, U.S. Patent Application 2003/0170881, U.S. Patent Application 2004/0018577, U.S. Patent Application 2005/0054078, and U.S. Patent Application 2006/0160164.

**E. Exemplary Kits** (not included in the present invention).

The present disclosure herein also can be adapted for use in a variety of kits for use on automated and semi-automated systems and platforms, e.g., commercially marketed by Abbott Laboratories (Abbott Park, IL) including, but not limited to, Abbott Laboratories' ARCHITECT®, AxSYM®, IMx®, PRISM®, and Quantum™ II instruments, Abbott Laboratories' commercial Point of Care (i-STAT™) electrochemical immunoassay system for performing sandwich immunoassays, as well as other platforms.

Such kits can comprise one or more of the immunodiagnostic reagents (e.g., antibodies) described herein. More specifically, if the kit is a kit for performing an immunoassay, the kit can optionally contain (1) at least one capture and detection antibody that bind to hBNP and together exhibit reduced cross-reactivity with human proBNP and human NT-proBNP; and (2) one or more instructions for performing the immunoassay. The immunodiagnostic reagents of the present disclosure can be included in such a test kit as a capture antibody, as a detection antibody or both as a capture antibody and a detection antibody. For example, antibody Clone 3 can be included in the kit as capture antibody and antibody AM1 can be included in the kit as a detection antibody. Alternatively, antibody Clone 3 can be included in the kit as a capture antibody and antibody 8.1 can be included in the kit as a detection antibody. Optionally, the kit can also contain at least one calibrator or control. Any calibrator or control can be included in the kit. Preferably, however, the calibrator or control is hBNP, especially SEQ ID NO: 4 described previously herein. Accordingly, the kits can comprise at least one calibrator, or at least one control, or a combination of at least one calibrator and at least one control.

Optionally the kits also can include quality control reagents (e.g., sensitivity panels, calibrators, and positive controls). Preparation of quality control reagents is well known in the art, and is described, e.g., on a variety of immunodiagnostic product insert sheets. Human BNP sensitivity panel members optionally can be prepared in varying amounts containing, e.g., known quantities of hBNP antigen ranging from "low" to "high", e.g., by spiking known quantities of the hBNP antigen into an appropriate assay buffer (e.g., a phosphate buffer). These sensitivity panel members optionally are used to establish assay performance characteristics, and further optionally are useful indicators of the integrity of the immunoassay kit reagents, and the standardization of assays. The hBNP antigen also can be employed as calibrators.

The antibodies provided in the kit can incorporate a detectable label, such as a fluorophore, radioactive moiety, enzyme, biotin/avidin label, chromophore, chemiluminescent label, or the like, or the kit may include reagents for labeling the antibodies or reagents for detecting the antibodies (e.g., detection antibodies) and/or for labeling the antigens or reagents for detecting the antigen. The antibodies, calibrators and/or controls can be provided in separate containers or pre-dispensed into an appropriate assay format, for example, into microtiter plates.

In yet another embodiment, the kit can comprise, either alone, with instructions, or with other aspects of the kit and kit components, an immunodiagnostic agent that comprises one or more antibodies selected from the group consisting of 106.3, BC203, Clone 3, M1, AM1, AM5, 201.3, AM8 and 8.1.

The kits can optionally include other reagents required to conduct a diagnostic assay or facilitate quality control evaluations, such as buffers, salts, enzymes, enzyme co-factors, substrates, detection reagents, and the like. Other components, such as buffers and solutions for the isolation and/or treatment of a test sample (e.g., pretreatment reagents), may also be included in the kit. The kit may additionally include one or more other controls. One or more of the components of the kit may be lyophilized and the kit may further comprise reagents suitable for the reconstitution of the lyophilized components.

The various components of the kit optionally are provided in suitable containers. As indicated above, one or more of the containers may be a microtiter plate. The kit further can include containers for holding or storing a sample (e.g., a container or cartridge for a blood or urine sample). Where appropriate, the kit may also optionally contain reaction vessels, mixing vessels and other components that facilitate the preparation of reagents or the test sample. The kit may also include one or more instruments for assisting with obtaining a test sample, such as a syringe, pipette, forceps, measured spoon, or the like.

The kit further can optionally include instructions for use, which may be provided in paper form or in computer-readable form, such as a disc, CD, DVD or the like.

Now by way of example, and not of limitation, examples of the present disclosure shall now be given.

**EXAMPLE 1: A Human B-type Natriuretic Peptide (hBNP) Sandwich Assay with <10% Cross-Reactivity to human proBNP**

For the ARCHITECT®-hBNP specific assay (hereinafter "Arc-hBNP") paramagnetic particles were coated with monoclonal antibody ("mAb") 3-631-436. This mAb binds to an amino acid sequence containing amino acids 13-18 on the hBNP peptide. Monoclonal antibodies produced by hybridoma cell line 3-631-436 are also referred interchangeably herein as "monoclonal antibody 3-631-436" or "Clone 3" and "BNP 3-631-436 ms". Additionally, murine hybridoma cell line 3-631-436 was deposited with the A.T.C.C. on December 21, 2004 and assigned A.T.C.C. Accession No. PTA-6476. The BNP 3-631-436 ms mAb was coated onto a paramagnetic particle (Polymer Laboratories, Amherst, MA) using the techniques described in U.S. Patent No. 6,162,902. Specifically, EDAC coupling was used. EDAC is generally used as a carboxyl activating agent for amide bonding with primary amines. In addition, it reacts with phosphate groups. It is used in peptide synthesis, crosslinking proteins to nucleic acids and in preparing immunoconjugates. The chemical formula for EDAC is 1-ethyl-3-(3-dimethylaminopropyl)carbodiimide, hydrochloride. EDAC is commercially available from Sigma-Aldrich, St. Louis, MO. Particles were washed and overcoated with BSA. These particles are used as the capture reagent in the assay during the first (1st) incubation with specimens.

Monoclonal antibody 106.3 L1 B24/H2 288 was conjugated to N10-(3-sulfopropyl)-N-(3-carboxypropyl)-acridinium-9-carboxamide active ester (also known as CPSP-Acridinium Ester) (Abbott Laboratories, Abbott Park, IL). This mAb binds to an amino acid sequence containing amino acids 5-13 on the hBNP peptide. Monoclonal antibodies produced by hybridoma cell line 106.3 L1 B24/H2 288 are also referred interchangeably herein as "monoclonal antibody 106.3 L1 B24/H2 288", "106.3AM1" and "AM1". Additionally, murine hybridoma cell line 106.3 L1 B24/H2 288 was deposited with the A.T.C.C. on September 20, 2005 and assigned A.T.C.C. Accession No. PTA-6987. Antibody 106.3AM1 labeled with acridinium is used in the assay during the second (2nd) incubation to detect the particle-bound hBNP peptide. The conjugation occurred by reaction of antibody 106.3AM1 with an activated acridinium-carboxamide ester.

Additional capture reagents (106.3AM1, BC203, 201.3, 8.1, M1) were prepared in a complimentary manner. These particles are used as the capture reagent in the assay during the first (1st) incubation with specimens. Additional detection reagents (8.1, M1, 201.3, BC203 and BNP 3-631-436 ms conjugates) were prepared in a complimentary manner to that described above. Antibody-acridinium conjugates were prepared using activated acridinium-carboxamide esters. The antibody-acridinium conjugates are used in the assay during the Second (2nd) incubation to detect the particle-bound hBNP peptide.

All immunoassays for hBNP were performed on an ARCHITECT® instrument (this instrument is described in U.S. Patent No. 5,468,646).

Full length hBNP calibrators (amino acid residues 1-32) were prepared by dilution of materials obtained from Peptides International, Inc. (Louisville, KY), into hBNP calibrator diluent (described in U.S. Patent Publication 2005/0014287). Human proBNP solutions (amino acid residues 1-108) were prepared by dilution of full-length human proBNP obtained from HyTest Ltd. (Turku, Finland) into the hBNP calibrator diluent. All human proBNP-spiked solutions used in % Recovery experiments were at concentrations equivalent to the hBNP calibrator concentrations on a molar basis.

Microparticles coated with a capture antibody in a Tris/BSA diluent were pipetted by the sampling probe into individual reaction vessels in the sampling center. An aliquot containing an hBNP calibrator or human proBNP-spiked solution was delivered to each reaction vessel containing the microparticles to form a reaction mixture. The reaction mixtures were incubated for approximately 4 minutes at a temperature of about 37 °C. After the incubation, the reaction mixtures were washed with ARCHITECT® line diluent to remove any of the hBNP or human proBNP peptide that was not captured. ARCHITECT® line diluent is commercially available from Abbott Laboratories, Abbott Park, Illinois.

The antibody-acridinium conjugates diluted to 150 ng/mL in a MES diluent were then dispensed into the reaction vessels and the resulting combinations were incubated for approximately 4 minutes at a temperature of about 37°C. After the incubation, the reaction vessels were washed with ARCHITECT® line diluent to remove the unbound materials.

A solution of hydrogen peroxide and then sodium hydroxide were added to the reaction vessels and the chemiluminescent signals were measured by the chemiluminescent microparticle immunoassay (CMIA) optical assembly of the ARCHITECT® instrument.

The ARCHITECT® system measures the acridinium signals which are typically measured in relative light units (hereinafter "rlu's"). Measurements were made in duplicate. The results shown in Tables 1a-1f below and in Figure 1 show the mean of the duplicate values of hBNP calibrators and typical calibration curves.

Table 1a. Calibration curves generated using BNP peptide as calibrators and selected BNP assays with anti-BNP (BNP 3-631-436 ms)mAb capture.

| Conjugate mAb | | 106.3 AM1 | BC203 | 8.1 | M1 |
|---|---|---|---|---|---|
| Concentration (pg/mL) | Concentration (pM)[1] | RLU | RLU | RLU | RLU |
| 0 | 0 | 1760 | 803 | 4159 | 12324 |
| 30 | 8.67 | 4078 | 1344 | 15055 | 13324 |
| 150 | 43.3 | 17137 | 4691 | 66922 | 14847 |

(continued)

| Conjugate mAb | | 106.3 AM1 | BC203 | 8.1 | M1 |
|---|---|---|---|---|---|
| Concentration (pg/mL) | Concentration (pM)[1] | RLU | RLU | RLU | RLU |
| 300 | 86.6 | 38823 | 11901 | 126474 | 22046 |
| 1000 | 288.7 | 238469 | 83506 | 437772 | 104940 |
| 2000 | 577.4 | 625439 | 269939 | 908089 | 330590 |
| [1]As used in this and all other Examples herein, "pM" refers to picomolar, i.e., pmol/L. | | | | | |

Table 1b. Calibration curves generated using BNP peptide as calibrators and selected BNP assays with anti-BNP (106.3AM1)mAb capture.

| Conjugate mAb | | BNP 3-631-436 ms | BC203 | 8.1 | M1 |
|---|---|---|---|---|---|
| Concentration (pg/mL) | Concentration (pM) | RLU | RLU | RLU | RLU |
| 0 | 0 | 3378 | 768 | 3504 | 9449 |
| 30 | 8.67 | 4134 | 1033 | 4095 | 9228 |
| 150 | 43.3 | 5970 | 3846 | 7958 | 15716 |
| 300 | 86.6 | 8583 | 8759 | 11874 | 25100 |
| 1000 | 288.7 | 39157 | 65520 | 37278 | 113669 |
| 2000 | 577.4 | 136114 | 223465 | 90574 | 355537 |

Table 1c. Calibration curves generated using BNP peptide as calibrators and selected BNP assays with anti-BNP (BC203)mAb capture.

| Conjugate mAb | | BNP 3-631-436 ms | 106.3AM1 |
|---|---|---|---|
| Concentration (pg/mL) | Concentration (pM) | RLU | RLU |
| 0 | 0 | 1362 | 1255 |
| 30 | 8.67 | 4993 | 7237 |
| 150 | 43.3 | 21697 | 35545 |
| 300 | 86.6 | 46450 | 80470 |
| 1000 | 288.7 | 194134 | 356040 |
| 2000 | 577.4 | 492653 | 876600 |

Table 1d. Calibration curves generated using BNP peptide as calibrators and selected BNP assays with anti-BNP (201.3)mAb capture.

| Conjugate mAb | | BNP 3-631-436 ms | 8.1 |
|---|---|---|---|
| Concentration (pg/mL) | Concentration (pM) | RLU | RLU |
| 0 | 0 | 7387 | 2983 |
| 30 | 8.67 | 8138 | 3372 |
| 150 | 43.3 | 8533 | 5938 |
| 300 | 86.6 | 11348 | 11060 |

(continued)

| Conjugate mAb | | BNP 3-631-436 ms | 8.1 |
|---|---|---|---|
| Concentration (pg/mL) | Concentration (pM) | RLU | RLU |
| 1000 | 288.7 | 32151 | 59565 |
| 2000 | 577.4 | 86700 | 213113 |

Table 1e. Calibration curves generated using BNP peptide as calibrators and selected BNP assays with anti-BNP (8.1)mAb capture.

| Conjugate mAb | | BNP 3-631-436 ms | 106.3AM1 | 201.3 |
|---|---|---|---|---|
| Concentration (pg/mL) | Concentration (pM) | RLU | RLU | RLU |
| 0 | 0 | 2528 | 2260 | 1853 |
| 30 | 8.67 | 8718 | 6794 | 2026 |
| 150 | 43.3 | 33750 | 26317 | 5252 |
| 300 | 86.6 | 71082 | 51849 | 12721 |
| 1000 | 288.7 | 263928 | 211266 | 119988 |
| 2000 | 577.4 | 605729 | 489568 | 433774 |

Table 1f. Calibration curves generated using BNP peptide as calibrators and selected BNP assays with anti-BNP (M1)mAb capture.

| Conjugate mAb | | BNP 3-631-436 ms | 106.3AM1 |
|---|---|---|---|
| Concentration (pg/mL) | Concentration (pM) | RLU | RLU |
| 0 | 0 | 2202 | 2542 |
| 30 | 8.67 | 2849 | 2821 |
| 150 | 43.3 | 4286 | 7182 |
| 300 | 86.6 | 7021 | 15115 |
| 1000 | 288.7 | 31134 | 77401 |
| 2000 | 577.4 | 84691 | 233153 |

Once the calibrator curves had been obtained, spiking studies were carried out to determine cross-reactivity with human proBNP. Spiking of proBNP was done at equimolar concentrations across the BNP calibrator range. **Figure 2** shows the mean % Recovery of spiked human proBNP peptide for selected antibody combinations. Tables 2a-2f show the mean readback concentration of human proBNP in pg/mL.

Table 2a. Detection of spiked proBNP (% Recovery) by selected BNP assays with anti-BNP(BNP 3-631-436 ms)mAb capture.

| Conjugate mAb | 106.3 AM1 | | BC203 | | 8.1 | | M1 | |
|---|---|---|---|---|---|---|---|---|
| Spike Conc. | Readback | Recovery | Recovery | Readback | Readback | Recovery | Readback | Recovery |
| (pg/mL) | (pg/mL) | (%) | (pg/mL) | (%) | (pg/mL) | (%) | (pg/mL) | (%) |
| 103 | 9.8 | 9.5 | -5.3 | -7.0 | 4.7 | 4.5 | 16.5 | 16.0 |
| 515 | 26.2 | 5.1 | 40.8 | 10.9 | 34.1 | 6.6 | -295.6 | -57.4 |

(continued)

| Conjugate mAb | 106.3 AM1 | | BC203 | | 8.1 | | M1 | |
|---|---|---|---|---|---|---|---|---|
| Spike Conc. | Readback | Recovery | Recovery | Readback | Readback | Recovery | Readback | Recovery |
| (pg/mL) | (pg/mL) | (%) | (pg/mL) | (%) | (pg/mL) | (%) | (pg/mL) | (%) |
| 1031 | 42.3 | 4.1 | 110.8 | 14.8 | 72.8 | 7.1 | 174.7 | 16.9 |
| 3436 | 176.9 | 5.1 | 482.5 | 19.3 | 280.2 | 8.2 | 945.5 | 27.5 |
| 6872 | 444.6 | 6.5 | 895.4 | 17.9 | 434.6 | 6.3 | 1745.7 | 25.4 |

Table 2b. Detection of spiked proBNP (% Recovery) by selected BNP assays with anti-BNP(106.3AM1)mAb capture.

| Conjugate mAb | BNP 3-631-436 ms | | BC203 | | 8.1 | | M1 | |
|---|---|---|---|---|---|---|---|---|
| Spike Conc. | Readback | Recovery | Readback | Recovery | Readback | Recovery | Readback | Recovery |
| (pg/mL) | | (%) | (pg/mL) | (%) | (pg/mL) | (%) | (pg/mL) | (%) |
| 103 | 46.6 | 45.2 | 117.2 | 113.8 | 68.1 | 66.2 | 263.7 | 256.0 |
| 515 | 73.2 | 14.2 | 189.7 | 36.8 | 151.4 | 29.4 | 424.8 | 82.5 |
| 1031 | 109.3 | 10.6 | 455.7 | 44.2 | 257.5 | 25.0 | 411.7 | 39.9 |
| 3436 | 809.2 | 23.6 | 1365.5 | 39.7 | 1079.4 | 31.4 | 1612.8 | 46.9 |
| 6872 | 1204.4 | 17.5 | 2829.0 | 41.2 | 2308.6 | 33.6 | 3461.4 | 50.4 |

Table 2c. Detection of spiked proBNP (% Recovery) by selected BNP assays with anti-BNP(BC203)mAb capture.

| Conjugate mAb | BNP 3-631-436 ms | | 106.3AM1 | |
|---|---|---|---|---|
| Spike Conc. | Readback | Recovery | Readback | Recovery |
| (pg/mL) | (pg/mL) | (%) | (pg/mL) | (%) |
| 103 | 14.1 | 13.7 | 9.7 | 9.5 |
| 515 | 51.9 | 10.1 | 51.4 | 10.0 |
| 1031 | 124.5 | 12.1 | 128.4 | 12.5 |
| 3436 | 444.6 | 12.9 | 481.2 | 14.0 |
| 6872 | 967.1 | 14.1 | 881.5 | 12.8 |

Table 2d. Detection of spiked proBNP (% Recovery) by selected BNP assays with anti-BNP(201.3)mAb capture.

| Conjugate mAb | BNP 3-631-436 ms | | 8.1 | |
|---|---|---|---|---|
| Spike Conc. | Readback | Recovery | Readback | Recovery |
| (pg/mL) | (pg/mL) | (%) | (pg/mL) | (%) |
| 103 | -38.1 | -37.0 | 31.2 | 30.3 |
| 515 | -13.7 | -2.7 | 52.4 | 10.2 |
| 1031 | 57.5 | 5.6 | 28.3 | 2.7 |
| 3436 | 0.4 | 0.0 | 59.3 | 1.7 |

(continued)

| Conjugate mAb | BNP 3-631-436 ms | | 8.1 | |
|---|---|---|---|---|
| Spike Conc. | Readback | Recovery | Readback | Recovery |
| (pg/mL) | (pg/mL) | (%) | (pg/mL) | (%) |
| 6872 | 96.3 | 1.4 | 210.1 | 3.1 |

Table 2e. Detection of spiked proBNP (% Recovery) by selected BNP assays with anti-BNP(8.1)mAb capture.

| Conjugate mAb | BNP 3-631-436 ms | | 106.3AM1 | | 201.3 | |
|---|---|---|---|---|---|---|
| Spike Conc. | Readback | Recovery | Readback | Recovery | Readback | Recovery |
| (pg/mL) | (pg/mL) | (%) | (pg/mL) | (%) | (pg/mL) | (%) |
| 103 | 9.6 | 9.4 | 6.9 | 6.7 | 74.4 | 72.3 |
| 515 | 64.2 | 12.5 | 46.5 | 9.0 | 23.8 | 4.6 |
| 1031 | 122.2 | 11.8 | 89.4 | 8.7 | -28.0 | -2.7 |
| 3436 | 465.4 | 13.5 | 297.5 | 8.7 | 0.0 | 0.0 |
| 6872 | 1099.9 | 16.0 | 690.2 | 10.0 | 0.0 | 0.0 |

Table 2f. Detection of spiked proBNP (% Recovery) by selected BNP assays with anti-BNP(M1)mAb capture.

| Conjugate mAb | BNP 3-631-436 ms | | 106.3AM1 | |
|---|---|---|---|---|
| Spike Conc. | Readback | Recovery | Readback | Recovery |
| (pg/mL) | | (%) | | (%) |
| 103 | 71.0 | 68.9 | 26.9 | 26.2 |
| 515 | 189.3 | 36.8 | 158.4 | 30.7 |
| 1031 | 257.5 | 25.0 | 246.0 | 23.9 |
| 3436 | 1058.4 | 30.8 | 746.8 | 21.7 |
| 6872 | 1673.7 | 24.4 | 1350.4 | 19.7 |

These data confirms assay configurations that are specific for human BNP with less than about 20%, or about 15%, and/or about 10% cross-reactivity with proBNP. For example, two assay configurations, BNP 3-631-436 ms/106.3 AMI and BNP 3-631-436 ms/8.1 show less than 10% cross-reactivity with proBNP and thus are BNP specific assays.

**EXAMPLE 2: A human pro B-type Natriuretic Peptide (human proBNP) Sandwich Assay with no Cross-Reactivity to hBNP**

For the ARCHITECT®-human proBNP specific assay (hereinafter "Arc-human proBNP") paramagnetic particles were coated with mAb 106.3AM1 described above. The 106.3AM1 mAb was coated onto a paramagnetic particle (Polymer Laboratories, Amherst, MA) as described above using the techniques described in U.S. Patent No. 6,162,902. These particles are used as the capture reagent in the assay during the first (1st) incubation with specimens.

Monoclonal antibody 18H5 was conjugated to acridinium (Abbott Laboratories, Abbott Park, IL). This mAb binds to an amino acid sequence containing amino acids 13-27 on the human proBNP peptide and thus binds outside the area of possible glycosylation. Monoclonal antibody 18H5 is commercially available from HyTest Ltd.(Turku, Finland). Antibody 18H5 labeled with acridinium is used in the assay during the second (2nd) incubation to detect the particle-bound human proBNP peptide. The conjugation occurred by reaction of antibody 18H5 with an activated acridinium-carboxamide ester.

All immunoassays for human proBNP were performed on an ARCHITECT® instrument. Human proBNP calibrators (amino acid residues 1-108) were prepared by dilution of materials obtained from HyTest Ltd. (Turku, Finland) in hBNP calibrator diluent. hBNP calibrators solutions from Example 1 were used as the hBNP-spiked solutions. All hBNP and human proBNP solutions utilized were of equal concentrations on a molar basis.

Microparticles coated with the 106.3AM1 capture antibody in a Tris/BSA diluent were pipetted by the sampling probe into individual reaction vessels in the sampling center. An aliquot containing a human proBNP calibrator or hBNP-spiked solution was delivered to each reaction vessel containing the microparticles to form a reaction mixture. The reaction mixtures were incubated for approximately 4 minutes at a temperature of about 37 °C. After the incubation, the reaction mixtures were washed with ARCHITECT® line diluent to remove any of the human proBNP or hBNP peptide that was not captured.

The 18H5 antibody-acridinium conjugate diluted to 150 ng/mL in a MES diluent was then dispensed into the reaction vessels and the resulting combinations were incubated for approximately 4 minutes at a temperature of about 37°C. After the incubation, the reaction vessels were washed with ARCHITECT® line diluent to remove the unbound materials.

A solution of hydrogen peroxide and then sodium hydroxide was added to the reaction vessels and the chemiluminescent signals were measured by the chemiluminescent microparticle immunoassay (CMIA) optical assembly of the ARCHITECT® instrument.

Measurements were made in triplicate. The results shown in Table 3 below and in **Figure 3** show the mean of the triplicate values of the human proBNP calibrators and a typical calibration curve.

Table 3. Calibration curve generated using proBNP peptide as calibrators and a proBNP-specific assay.

| μParticle mAb Conjugate mAb | | 106.3AM1 18H5 |
| --- | --- | --- |
| Concentration (pg/mL) | Concentration (pM) | RLU |
| 0 | 0 | 951 |
| 103 | 8.67 | 2809 |
| 515 | 43.3 | 10806 |
| 1031 | 86.6 | 23933 |
| 3436 | 288.7 | 83741 |
| 6872 | 577.4 | 181248 |

Specifically, the results in Table 4 show mean readback concentration of hBNP in pg/mL and the % Recovery of spiked hBNP peptide.

Table 4. Detection of spiked BNP by proBNP-specific assay.

| μParticle mAb Conjugate mAb | 106.3 AMI 18H5 | |
| --- | --- | --- |
| Spike Conc. (pg/mL) | Readback (pg/mL) | Recovery (%) |
| 150 | -0.7 | -0.5 |
| 300 | -1.4 | -0.5 |
| 1000 | 0.1 | 0.0 |
| 2000 | -0.1 | 0.0 |

Thus, as shown above, the assay with 106.3 AM1 and 18 H5 detects only human proBNP with no cross-reactivity with hBNP.

**EXAMPLE 3: Measurement of the human proBNP/hBNP Ratio in Clinical Samples**

Twenty EDTA plasma specimens were obtained from ARUP Laboratories (Salt Lake City, UT). The samples were shipped on dry ice and stored at -70 °C before use. The samples were obtained from both male and female subjects of varying ages for whom a physician had requested a BNP test.

ARCHITECT®-hBNP specific measurements were made as described above using the BNP 3-631-436 ms

24

microparticle/106.3AM1 acridinium conjugate combination. ARCHITECT®-human proBNP specific measurements were made as described above using the 106.3AM1 uparticle/18H5 acridinium conjugate combination. All immunoassays for hBNP and human proBNP were performed on the ARCHITECT® instrument using the hBNP calibrators (amino acid residues 1-32) and human proBNP calibrators (amino acid residues 1-108) prepared above.

All hBNP and human proBNP calibrators were measured in duplicate using the analyte-specific kit and fit using a point-to-point data reduction method to generate a calibration curve for each analyte. The concentrations of hBNP and proBNP in each patient specimen were determined from single measurements read off the respective calibration curve. Readback values for hBNP and human proBNP concentrations in each patient specimen are shown in Table 5 below. Specifically, the results in Table 5 are depicted in pg/mL or picomolar (pM) hBNP or human proBNP in the patient specimen.

Table 5. Ratios of proBNP/BNP or BNP/proBNP in Clinical Samples

| | Weight Measure | | | | Molar Measure | | | |
|---|---|---|---|---|---|---|---|---|
| | pg/mL | | Weight Ratio | | pM | | Molar Ratio | |
| Sample | BNP | proBNP | BNP/pro | pro/BNP | BNP | proBNP | BNP/pro | proBNP |
| ARUP01 | 58.1 | 432.5 | 0.134 | 7.44 | 16.8 | 36.3 | 0.462 | 2.17 |
| ARUP02 | 130.4 | 986.0 | 0.132 | 7.56 | 37.6 | 82.8 | 0.454 | 2.20 |
| ARUP03 | 34.0 | 308.0 | 0.110 | 9.07 | 9.8 | 25.9 | 0.379 | 2.64 |
| ARUP04 | 148.1 | 708.8 | 0.209 | 4.79 | 42.8 | 59.6 | 0.718 | 1.39 |
| ARUP05 | 307.0 | 2283.6 | 0.134 | 7.44 | 88.6 | 191.9 | 0.462 | 2.16 |
| ARUP06 | 48.7 | 331.2 | 0.147 | 6.80 | 14.1 | 27.8 | 0.505 | 1.98 |
| ARUP07 | 81.9 | 577.2 | 0.142 | 7.05 | 23.6 | 48.5 | 0.487 | 2.05 |
| ARUP08 | 3.7 | 73.1 | 0.051 | 19.72 | 1.1 | 6.1 | 0.174 | 5.74 |
| ARUP09 | 33.0 | 205.4 | 0.161 | 6.22 | 9.5 | 17.3 | 0.552 | 1.81 |
| ARUP10 | 186.9 | 1167.4 | 0.160 | 6.25 | 53.9 | 98.1 | 0.550 | 1.82 |
| ARUP11 | 547.7 | 3329.4 | 0.165 | 6.08 | 158.1 | 279.7 | 0.565 | 1.77 |
| ARUP12 | 68.6 | 377.5 | 0.182 | 5.50 | 19.8 | 31.7 | 0.624 | 1.60 |
| ARUP13 | 781.7 | 5244.2 | 0.149 | 6.71 | 225.7 | 440.6 | 0.512 | 1.95 |
| ARUP14 | 119.0 | 1042.7 | 0.114 | 8.76 | 34.3 | 87.6 | 0.392 | 2.55 |
| ARUP15 | 60.2 | 714.8 | 0.084 | 11.87 | 17.4 | 60.1 | 0.290 | 3.45 |
| ARUM16 | 942.0 | 5394.6 | 0.175 | 5.73 | 272.0 | 453.3 | 0.600 | 1.67 |
| ARUP17 | 591.6 | 4862.2 | 0.122 | 8.22 | 170.8 | 408.5 | 0.418 | 2.39 |
| ARUP18 | 255.4 | 1531.1 | 0.167 | 6.00 | 73.7 | 128.6 | 0.573 | 1.74 |
| ARUP19 | 197.2 | 1746.1 | 0.113 | 8.85 | 56.9 | 146.7 | 0.388 | 2.58 |
| ARUP20 | 36.5 | 333.0 | 0.110 | 9.13 | 10.5 | 28.0 | 0.376 | 2.66 |

The molar and weight ratios of human proBNP to BNP or hBNP to human proBNP were also determined. The molar ratios of proBNP/BNP (See, Table 5 above) ranged from 1.39 to 5.74. The molar ratios of BNP/proBNP ranged from 0.17 to 0.72. An approximate four-fold range in ratios was noted. The weight ratios of proBNP/BNP ranged from 4.79 to 19.72 and for BNP/proBNP ranged from 0.051 to 0.209. Again, an approximated four-fold range in ratios was noted.

**EXAMPLE 4: Measurement of the Human ProBNP/hBNP Molar and Weight Ratios in Clinical Samples**

Seventy three (73) HF plasma specimens with designated New York Heart Association Classifications I-IV were obtained from ProMedDx, LLC (Norton, MA). The samples were shipped on dry ice and stored at -70 °C before use.
ARCHITECT®-hBNP specific measurements were made as described above using the Clone 3 microparticle/ 106.3AM1 acridinium conjugate combination. ARCHITECT®-human proBNP specific measurements were made as described above using the 106.3AM1 microparticle/18H5 acridinium conjugate combination. All immunoassays for hBNP and human proBNP were performed on the ARCHITECT® instrument using the hBNP calibrators (amino acid residues 1-32) and human proBNP calibrators (amino acid residues 1-108) prepared above.

All hBNP and human proBNP calibrators were measured in duplicate using the analyte-specific kit and fit using a point-to-point data reduction method to generate a calibration curve for each analyte. The concentrations of hBNP and human proBNP in each patient specimen were determined from single measurements read off the respective calibration curve. Readback values for hBNP and human proBNP concentrations in each patient specimen are shown in Tables 6 A and B below. The molar and weight ratios of human proBNP/hBNP and hBNP/human proBNP are also shown in Tables 6A and B. **Figure 4** is the plot of hBNP versus human proBNP concentrations determined showing a general correlation between the values. Specifically, the results in **Figure 4** are depicted in pM hBNP or human proBNP in the patient specimens. The average value of the human proBNP/hBNP molar and weight ratios for each NYHA Classification are depicted in Tables 7A and B. NYHA class III and IV patients are grouped together because of severity of disease and the small (n=3) number of NYHA IV samples tested.

Table 6A

| NYHA Class I | | | | I NYHA Class II | | | | I NYHA Class III/IV | | | |
| pg/mL | | Weight Ratio | | pg/mL | | Weight Ratio | | pg/mL | | Weight Ratio | |
| BNP | proBNP | BNP/pro | pro/BNP | BNP | proBNP | BNP/pro | pro/BNP | BNP | proBNP | BNP/pro | pro/BNP |
|---|---|---|---|---|---|---|---|---|---|---|---|
| 5.1 | 302.1 | 0.017 | 59.22 | 5.6 | 674.8 | 0.008 | 119.83 | 16.3 | 570.6 | 0.029 | 34.93 |
| 4.8 | 149.4 | 0.032 | 31.38 | 37.6 | 1054.0 | 0.036 | 28.00 | 90.9 | 3167.3 | 0.029 | 34.86 |
| 128.2 | 1640.7 | 0.078 | 12.80 | 31.3 | 564.5 | 0.055 | 18.05 | 18.2 | 685.6 | 0.027 | 37.72 |
| 10.5 | 1476.6 | 0.007 | 140.88 | 285.31 | 3618.6 | 0.079 | 12.68 | 4.3 | 241.3 | 0.018 | 55.50 |
| 55.0 | 1210.9 | 0.045 | 22.03 | 11.4 | 476.0 | 0.024 | 41.82 | 29.3 | 1193.8 | 0.025 | 40.71 |
| 44.1 | 1236.0 | 0.036 | 28.03 | 68.1 | 1814.1 | 0.038 | 26.65 | 48.5 | 464.2 | 0.104 | 9.57 |
| 34.4 | 510.5 | 0.067 | 14.82 | 30.5 | 3308.9 | 0.009 | 108.6 | 168.0 | 1505.3 | 0.112 | 8.96 |
| 115.2 | 1159.0 | 0.099 | 10.06 | 60.6 | 1310.6 | 0.046 | 21.64 | 552.7 | 6198.7 | 0.089 | 11.21 |
| 28.8 | 668.9 | 0.043 | 23.20 | 19.6 | 265.8 | 0.074 | 13.54 | 137.6 | 1996.1 | 0.069 | 14.51 |
| 154.5 | 3059.4 | 0.050 | 19.80 | 35.8 | 483.8 | 0.074 | 13.51 | 25.9 | 410.7 | 0.063 | 15.84 |
| | | | | 346.7 | 3845.9 | 0.090 | 11.09 | 10.2 | 237.4 | 0.043 | 23.17 |
| | | | | 230.4 | 3109.6 | 0.074 | 13.50 | 65.1 | 1586.6 | 0.041 | 24.36 |
| | | | | 40.2 | 619.6 | 0.065 | 15.42 | 136.0 | 1799.9 | 0.076 | 13.23 |
| | | | | 27.2 | 733.1 | 0.037 | 26.97 | 90.8 | 1670.4 | 0.054 | 18.39 |
| | | | | 33.6 | 578.6 | 0.058 | 17.23 | 107.4 | 2367.1 | 0.045 | 22.05 |
| | | | | 390.4 | 5912.3 | 0.066 | 15.14 | 29.7 | 733.3 | 0.040 | 24.70 |
| | | | | 12.91 | 195.9 | 0.066 | 15.17 | 172.2 | 3504.0 | 0.049 | 20.35 |
| | | | | 21.1 | 314.6 | 0.067 | 14.93 | 84.2 | 2083.1 | 0.040 | 24.74 |
| | | | | 7.2 | 179.0 | 0.040 | 24.78 | 44.2 | 1162.2 | 0.038 | 26.32 |
| | | | | 71.9 | 1089.9 | 0.066 | 15.16 | 40.4 | 786.1 | 0.051 | 19.48 |
| | | | | 55.5 | 1159.2 | 0.048 | 20.88 | 99.1 | 2328.9 | 0.043 | 23.50 |
| | | | | 6.5 | 118.2 | 0.055 | 18.18 | 30.0 | 714.8 | 0.042 | 23.83 |
| | | | | 8.2 | 221.0 | 0.037 | 26.86 | 69.6 | 1013.4 | 0.069 | 14.57 |

| NYHA Class I | | | | I NYHA Class II | | | | I NYHA Class III/IV | | | |
|---|---|---|---|---|---|---|---|---|---|---|---|
| pg/mL | | Weight Ratio | | pg/mL | | Weight Ratio | | pg/mL | | Weight Ratio | |
| BNP | proBNP | BNP/pro | pro/BNP | BNP | proBNP | BNP/pro | pro/BNP | BNP | proBNP | BNP/pro | pro/BNP |
| | | | | 94.4 | 2265.6 | 0.042 | 24.01 | 18.2 | 268.8 | 0.068 | 14.78 |
| | | | | 175.7 | 1621.8 | 0.108 | 9.23 | 189.7 | 2717.7 | 0.070 | 14.33 |
| | | | | 192.0 | 1916.4 | 0.100 | 9.98 | 100.7 | 3810.9 | 0.026 | 37.84 |
| | | | | 80.1 | 881.4 | 0.091 | 11.01 | 47.9 | 701.5 | 0.068 | 14.65 |
| | | | | | | | | 70.0 | 1312.2 | 0.053 | 18.75 |
| | | | | | | | | 47.7 | 674.5 | 0.071 | 14.15 |
| | | | | | | | | 34.8 | 623.9 | 0.056 | 17.92 |
| | | | | | | | | 231.9 | 2944.1 | 0.079 | 12.70 |
| | | | | | | | | 107.7 | 1343.8 | 0.080 | 12.48 |
| | | | | | | | | 79.1 | 1593.9 | 0.050 | 20.16 |
| | | | | | | | | 41.0 | 1374.1 | 0.030 | 33.54 |
| | | | | | | | | 361.6 | 6792.8 | 0.053 | 18.79 |
| | | | | | | | | 178.3 | 3417.4 | 0.052 | 19.17 |

Table 6B

| NYHA Class I | | | | NYHA Class II | | | | NYHA Class III/IV | | | |
|---|---|---|---|---|---|---|---|---|---|---|---|
| pM | | Molar Ratio | | pM | | Molar Ratio | | pM | | Molar Ratio | |
| BNP | proBNP | BNP/pro | pro/BNP | BNP | proBNP | BNP/pro | pro/BNP | BNP | proBNP | BNP/pro | pro/BNP |
| 1.5 | 25.4 | 0.058 | 17.24 | 1.6 | 56.7 | 0.029 | 34.87 | 4.7 | 47.9 | 0.098 | 10.17 |
| 1.4 | 12.6 | 0.109 | 9.13 | 10.9 | 88.6 | 0.123 | 8.15 | 26.2 | 266.1 | 0.099 | 10.15 |
| 37.0 | 137.9 | 0.268 | 3.73 | 9.0 | 47.4 | 0.190 | 5.25 | 5.2 | 57.6 | 0.091 | 10.98 |
| 3.0 | 124.1 | 0.024 | 41.00 | 82.4 | 304.0 | 0.271 | 3.69 | 1.3 | 20.3 | 0.062 | 16.15 |
| 15.9 | 101.7 | 0.156 | 6.41 | 3.3 | 40.0 | 0.082 | 12.17 | 8.5 | 100.3 | 0.084 | 11.85 |
| 12.7 | 103.8 | 0.123 | 8.16 | 19.6 | 152.4 | 0.129 | 7.76 | 14.0 | 39.0 | 0.359 | 2.79 |
| 9.9 | 42.9 | 0.232 | 4.31 | 8.8 | 278.0 | 0.032 | 31.61 | 48.5 | 126.5 | 0.383 | 2.61 |
| 33.3 | 97.4 | 0.342 | 2.93 | 17.5 | 110.1 | 0.159 | 6.30 | 159.6 | 520.8 | 0.306 | 3.26 |
| 8.3 | 56.2 | 0.148 | 6.75 | 5.7 | 22.3 | 0.254 | 3.94 | 39.7 | 167.7 | 0.237 | 4.22 |
| 44.6 | 257.1 | 0.174 | 5.76 | 10.3 | 40.6 | 0.254 | 3.93 | 7.5 | 34.5 | 0.217 | 4.61 |
| | | | | 100.1 | 323.1 | 0.310 | 3.23 | 3.0 | 19.9 | 0.148 | 6.74 |
| | | | | 66.5 | 261.3 | 0.255 | 3.93 | 18.8 | 133.3 | 0.141 | 7.09 |
| | | | | 11.6 | 52.1 | 0.223 | 4.49 | 39.3 | 151.2 | 0.260 | 3.85 |
| | | | | 7.8 | 61.6 | 0.127 | 7.85 | 26.2 | 140.3 | 0.187 | 5.35 |
| | | | | 9.7 | 48.6 | 0.199 | 5.01 | 31.0 | 198.9 | 0.156 | 6.42 |
| | | | | 112.7 | 496.8 | 0.227 | 4.41 | 8.6 | 61.6 | 0.139 | 7.19 |
| | | | | 3.7 | 16.5 | 0.226 | 4.42 | 49.7 | 294.4 | 0.169 | 5.92 |
| | | | | 6.1 | 26.4 | 0.230 | 4.35 | 24.3 | 175.0 | 0.139 | 7.20 |
| | | | | 2.1 | 15.0 | 0.139 | 7.21 | 12.7 | 97.6 | 0.131 | 7.66 |
| | | | | 20.8 | 91.6 | 0.227 | 4.41 | 11.7 | 66.0 | 0.176 | 5.67 |
| | | | | 16.0 | 97.4 | 0.165 | 6.08 | 28.6 | 195.7 | 0.146 | 6.84 |
| | | | | 1.9 | 9.9 | 0.189 | 5.29 | 8.7 | 60.1 | 0.144 | 6.93 |
| | | | | 2.4 | 18.6 | 0.128 | 7.82 | 20.1 | 85.1 | 0.236 | 4.24 |

(continued)

| NYHA Class I | | | | NYHA Class II | | | | NYHA Class III/IV | | | |
| pM | | Molar Ratio | | pM | | Molar Ratio | | pM | | Molar Ratio | |
| BNP | proBNP | BNP/pro | pro/BNP | BNP | proBNP | BNP/pro | pro/BNP | BNP | proBNP | BNP/pro | pro/BNP |
|---|---|---|---|---|---|---|---|---|---|---|---|
| | | | | 27.2 | 190.4 | 0.143 | 6.99 | 5.3 | 22.6 | 0.233 | 4.30 |
| | | | | 50.7 | 136.3 | 0.372 | 2.69 | 54.8 | 228.3 | 0.240 | 4.17 |
| | | | | 55.4 | 161.0 | 0.344 | 2.90 | 29.1 | 320.2 | 0.091 | 11.01 |
| | | | | 23.1 | 74.1 | 0.312 | 3.20 | 13.8 | 58.9 | 0.235 | 4.26 |
| | | | | | | | | 20.2 | 110.2 | 0.183 | 5.46 |
| | | | | | | | | 13.8 | 56.7 | 0.243 | 4.12 |
| | | | | | | | | 10.1 | 52.4 | 0.192 | 5.22 |
| | | | | | | | | 66.9 | 247.4 | 0.271 | 3.70 |
| | | | | | | | | 31.1 | 112.9 | 0.275 | 3.63 |
| | | | | | | | | 22.8 | 133.9 | 0.170 | 5.87 |
| | | | | | | | | 11.8 | 115.5 | 0.102 | 9.76 |
| | | | | | | | | 104.4 | 570.7 | 0.183 | 5.47 |
| | | | | | | | | 51.5 | 287.1 | 0.179 | 5.58 |

Table 7A

| Average Weight Ratio | NYHA Class | | |
|---|---|---|---|
| | I (n=10) | II (n=27) | III/IV (n=36) |
| BNP/proBNP | 0.048 | 0.058 | 0.054 |
| ProBNP/BNP | 36.22 | 25.70 | 21.99 |

Table 7B

| Average Molar Ratio | NYHA Class | | |
|---|---|---|---|
| | I (n=10) | II (n=27) | III/IV (n=36) |
| BNP/proBNP | 0.163 | 0.198 | 0.186 |
| ProBNP/BNP | 10.54 | 7.48 | 6.40 |

A correlation was found between the ratio of human proBNP and hBNP in subjects having heart failure designated in New York Heart Association Classifications I-IV, namely a decrease in human proBNP/hBNP ratio (molar or weight) was observed with an increase in disease severity as measured by clinical indicia (i.e., an increase in NYHA class).

One skilled in the art would readily appreciate that the present disclosure is well adapted to carry out the objects and obtain the ends and advantages mentioned, as well as those inherent therein. The molecular complexes and the methods, procedures, treatments, molecules, specific compounds described herein are presently representative of preferred embodiments, are exemplary, and are not intended as limitations on the scope of the disclosure herein. It will be readily apparent to one skilled in the art that varying substitutions and modifications may be made to the disclosure herein without departing from the scope and spirit.

All patents and publications mentioned in the specification are indicative of the levels of those skilled in the art to which the disclosure pertains.

The disclosure illustratively described herein suitably may be practiced in the absence of any element or elements, limitation or limitations which is not specifically disclosed herein. Thus, for example, in each instance herein any of the terms "comprising," "consisting essentially of" and "consisting of" may be replaced with either of the other two terms. The terms and expressions which have been employed are used as terms of description and not of limitation, and there is no intention that in the use of such terms and expressions of excluding any equivalents of the features shown and described or portions thereof.

**Claims**

1. An *in vitro* method of determining the severity of cardiovascular disease in a subject, the *in vitro* method comprising the steps of:

   (a) determining the amount of hBNP in a test sample according to an immunoassay for quantifying the amount of hBNP present;
   (b) determining the amount of human proBNP in said sample;
   (c) determining the molar ratio or weight ratio of the amount of human proBNP to the amount of hBNP in said sample; and
   (d) correlating the molar ratio or weight ratio with severity of cardiovascular disease in the subject wherein if the ratio is lower than a predetermined level the subject is determined to have increased severity of cardiovascular disease, and if the ratio is higher than a predetermined level the subject is determined to have reduced severity of cardiovascular disease;
   wherein said immunoassay for determining the amount of hBNP has reduced cross-reactivity with any human proBNP present in the test sample and comprises the steps of:

   (I) contacting at least one capture antibody that binds to hBNP and that has been immobilized onto a solid phase to produce an immobilized antibody with said test sample to form a first mixture comprising an at least one capture antibody-hBNP complex, wherein said capture antibody comprises one or more antibodies having an equilibrium dissociation constant ($K_D$) of between about $3.0 \times 10^{-7}$ and about $1.0 \times 10^{-13}$ M;
   (II) contacting said first mixture comprising the at least one capture antibody- hBNP complex with at least one detection antibody that binds to hBNP and that has been conjugated to a detectable label to form a second mixture comprising at least one capture antibody-hBNP-at least one detection antibody complex, wherein the detection antibody comprises one or more antibodies having an equilibrium dissociation constant ($K_D$) of between about $3.0 \times 10^{-7}$ and about $1.0 \times 10^{-13}$ M; and
   (III) determining the amount of the at least one capture antibody-hBNP-at least one detection antibody complex formed in step (II) by detecting the detectable label as a measure of the amount of hBNP contained in the test sample,

   wherein the at least one capture antibody and the at least one detection antibody, when used together, exhibit a cross-reactivity of less than about 20% with any human proBNP present in the test sample.

2. The *in vitro* method of claim 1, wherein the cardiovascular disease is selected from the group consisting of coronary artery disease, peripheral vascular disease, hypertension, myocardial infarction and heart failure.

3. An *in vitro* method of monitoring the progression of cardiovascular disease in a subject, the *in vitro* method comprising the steps of:

   (a) determining the amount of hBNP in the test sample according to an immunoassay for quantifying the amount of hBNP;
   (b) determining the amount of human proBNP in said sample;
   (c) determining the molar ratio or weight ratio of the amount of human proBNP to the amount of hBNP in said sample; and
   (d) correlating the molar ratio or weight ratio with progression of disease in the subject wherein the ratio is lower as compared to that in an earlier test sample from the subject with progression, and the ratio is unaltered or higher as compared to that in an earlier test sample from the subject with non-progression or improvement of cardiovascular disease;
   wherein said immunoassay for determining the amount of hBNP has reduced cross-reactivity with any human proBNP present in the test sample and comprises the steps of:

(I) contacting at least one capture antibody that binds to hBNP and that has been immobilized onto a solid phase to produce an immobilized antibody with said test sample to form a first mixture comprising an at least one capture antibody-hBNP complex, wherein said capture antibody comprises one or more antibodies having an equilibrium dissociation constant ($K_D$) of between about $3.0 \times 10^{-7}$ and about $1.0 \times 10^{-13}$ M;

(II) contacting said first mixture comprising the at least one capture antibody- hBNP complex with at least one detection antibody that binds to hBNP and that has been conjugated to a detectable label to form a second mixture comprising at least one capture antibody-hBNP-at least one detection antibody complex, wherein the detection antibody comprises one or more antibodies having an equilibrium dissociation constant ($K_D$) of between about $3.0 \times 10^{-7}$ and about $1.0 \times 10^{-13}$ M; and

(III) determining the amount of the at least one capture antibody-hBNP at least one detection antibody complex formed in step (II) by detecting the detectable label as a measure of the amount of hBNP contained in the test sample,

wherein the at least one capture antibody and the at least one detection antibody, when used together, exhibit a cross-reactivity of less than about 20% with any human proBNP present in the test sample.

4. The *in vitro* method of claim 3, wherein said monitoring is done following treatment for the cardiovascular disease.

5. An *in vitro* method of identifying a subject that would benefit from natriuretic peptide derivative treatment for cardiovascular disease, the *in vitro* method comprising the steps of:

(a) determining the amount of human BNP in a test sample from a subject that exhibits one or more clinical indicia associated with cardiovascular disease, which determination is carried out with an immunoassay for quantifying the amount of hBNP;

(b) determining the amount of human proBNP in said sample;

(c) determining the molar ratio or weight ratio of the amount of human proBNP to the amount of hBNP in said sample;

(d) determining whether the molar ratio or weight ratio determined in step (c) is higher or lower than a predetermined level; and

(e) identifying whether the subject would benefit from natriuretic peptide derivative treatment based on the determination in step (d), wherein if the ratio is lower as compared to the predetermined level, the subject is identified as a subject that would not benefit from natriuretic peptide derivative treatment and further wherein, if the ratio is higher than a predetermined level, then the subject is identified as a subject that would benefit from natriuretic peptide derivative treatment;

wherein said immunoassay for determining the amount of human BNP has reduced cross-reactivity with any human proBNP present in the test sample and comprises the steps of:

(I) contacting at least one capture antibody that binds to hBNP and that has been immobilized onto a solid phase to produce an immobilized antibody with said test sample to form a first mixture comprising an at least one capture antibody-hBNP complex, wherein said capture antibody comprises one or more antibodies having an equilibrium dissociation constant ($K_D$) of between about $3.0 \times 10^{-7}$ and about $1.0 \times 10^{-13}$ M;

(II) contacting said first mixture comprising the at least one capture antibody- hBNP complex with at least one detection antibody that binds to hBNP and that has been conjugated to a detectable label to form a second mixture comprising at least one capture antibody-hBNP-at least one detection antibody complex, wherein the detection antibody comprises one or more antibodies having an equilibrium dissociation constant ($K_D$) of between about $3.0 \times 10^{-7}$ and about $1.0 \times 10^{-13}$ M; and

(III) determining the amount of the at least one capture antibody-hBNP-at least one detection antibody complex formed in step (II) by detecting the detectable label as a measure of the amount of hBNP contained in the test sample,

wherein the at least one capture antibody and the at least one detection antibody, when used together, exhibit a cross-reactivity of less than about 20% with any human proBNP present in the test sample.

6. The *in vitro* method of claim 5, wherein the natriuretic peptide derivative is nesiritide.

7. An *in vitro* method of determining if a subject has suffered a cardiovascular complication as a result of administration to said subject of one or more pharmaceutical compositions, the *in vitro* method comprising the steps of:

(a) determining the amount of human BNP in a test sample before the subject has been administered one or more pharmaceutical compositions with an immunoassay for quantifying the amount of hBNP present in a test sample;

(b) determining the amount of human proBNP in said sample;

(c) determining the molar ratio or weight ratio of the amount of human proBNP to the amount of hBNP in said sample;

(d) determining the amount of human BNP in a second test sample from the subject after the subject has been administered one or more pharmaceutical compositions which determination is carried out with said immunoassay;

(e) determining the amount of human proBNP in said second test sample;

(f) determining the molar ratio or weight ratio of the amount of human proBNP to the amount of hBNP in said second test sample; and

(g) comparing the molar ratio or weight ratio determined in step (c) with the molar or weight ratio in step (f), wherein if the molar ratio or weight ratio determined in step (c) is unchanged when compared to the molar ratio or weight ratio determined in step (f), then the subject is determined not to have suffered a cardiovascular complication as a result of the administration of one or more pharmaceutical compositions and further wherein if the molar ratio or weight ratio determined in step (c) is changed when compared to the molar ratio or weight ratio determined in step (f), then the subject is determined to have suffered a cardiovascular complication as a result of the administration of one or more pharmaceutical compositions;

wherein said immunoassay for determining the amount of human BNP has reduced cross-reactivity with any human proBNP present in the test sample and comprises the steps of:

(I) contacting at least one capture antibody that binds to hBNP and that has been immobilized onto a solid phase to produce an immobilized antibody with said test sample to form a first mixture comprising an at least one capture antibody-hBNP complex, wherein said capture antibody comprises one or more antibodies having an equilibrium dissociation constant ($K_D$) of between about $3.0 \times 10^{-7}$ and about $1.0 \times 10^{-13}$ M;

(II) contacting said first mixture comprising the at least one capture antibody- hBNP complex with at least one detection antibody that binds to hBNP and that has been conjugated to a detectable label to form a second mixture comprising at least one capture antibody-hBNP-at least one detection antibody complex, wherein the detection antibody comprises one or more antibodies having an equilibrium dissociation constant ($K_D$) of between about $3.0 \times 10^{-7}$ and about $1.0 \times 10^{-13}$ M; and

(III) determining the amount of the at least one capture antibody-hBNP-at least one detection antibody complex formed in step (II) by detecting the detectable label as a measure of the amount of hBNP contained in the test sample,

wherein the at least one capture antibody and the at least one detection antibody, when used together, exhibit a cross-reactivity of less than about 20% with any human proBNP present in the test sample.

**Patentansprüche**

1. Ein in *vitro-Verfahren* zur Bestimmung der Schwere einer kardiovaskulären Erkrankung in einem Subjekt, wobei das in *vitro-Verfahren* die folgenden Schritte umfasst:

(a) Bestimmen der Menge von hBNP in einer Testprobe gemäß einem Immunoassay zur Quantifizierung der vorhandenen Menge an hBNP;

(b) Bestimmen der Menge an menschlichem proBNP in der Probe;

(c) Bestimmen des molaren Verhältnisses oder des Gewichtsverhältnisses von der Menge an menschlichem proBNP zu der Menge an hBNP in der Probe; und

(d) Korrelieren des molaren Verhältnisses oder des Gewichtsverhältnisses mit der Schwere der kardiovaskulären Erkrankung in dem Subjekt, worin, wenn das Verhältnis geringer ist als ein vorbestimmter Level, ermittelt wird, dass das Subjekt eine erhöhte Schwere der kardiovaskulären Erkrankung hat, und wenn das Verhältnis höher als ein vorbestimmter Level ist, ermittelt wird, dass das Subjekt eine verminderte Schwere der kardiovaskulären Erkrankung hat;

worin der Immunoassay zur Bestimmung der Menge von hBNP eine verminderte Kreuz-Reaktivität mit jeglichem menschlichen proBNP hat, das in der Testprobe vorhanden ist, und die folgenden Schritte umfasst:

(I) In-Kontakt-bringen von mindestens einem Einfang-Antikörper, der an hBNP bindet, und der auf eine feste Phase immobilisiert wurde, um einen immobilisierten Antikörper zu erzeugen, mit der Testprobe, um eine erste Mischung zu bilden, die mindestens einen Einfang-Antikörper-hBNP-Komplex umfasst, worin der Einfang-Antikörper einen oder mehrere Antikörper mit einer Gleichgewichtsdissoziationskonstante ($K_D$) von zwischen ungefähr $3{,}0 \times 10^{-7}$ und ungefähr $1{,}0 \times 10^{-13}$ M umfasst;

(II) In-Kontakt-bringen der ersten Mischung umfassend den mindestens einen Einfang-Antikörper-hBNP-Komplex mit mindestens einem Detektionsantikörper, der an hBNP bindet und der an einen detektierbaren Marker konjugiert wurde, um eine zweite Mischung zu bilden, die mindestens einen Einfang-Antikörper-hBNPmindestens ein Detektionsantikörper-Komplex umfasst, worin der Detektionsantikörper einen oder mehrere Antikörper mit einer Gleichgewichtsdissoziationskonstante ($K_D$) von zwischen ungefähr $3{,}0 \times 10^{-7}$ und ungefähr $1{,}0 \times 10^{-13}$ M umfasst; und

(III) Bestimmen der Menge des mindestens einen Einfang-Antikörper-hBNP-mindestens ein Detektionsantikörper-Komplexes, der in Schritt (II) gebildet wurde, durch Detektieren des detektierbaren Markers als ein Maß für die Menge an hBNP, die in der Testprobe enthalten ist,

worin der mindestens eine Einfang-Antikörper und der mindestens eine Detektionsantikörper, wenn sie zusammen verwendet werden, eine Kreuz-Reaktivität von weniger als ungefähr 20% zeigen mit jeglichem menschlichen proBNP, das in der Testprobe vorhanden ist.

2.  Das in *vitro-Verfahren* gemäß Anspruch 1, worin die kardiovaskulären Erkrankung gewählt ist aus der Gruppe bestehend aus Koronararterienerkrankung, peripherer Gefäßerkrankung, Bluthochdruck, Myokardinfarkt und Herzinfarkt.

3.  Ein in *vitro-Verfahren* zur Überwachung des Fortschreitens einer kardiovaskulären Erkrankung in einem Subjekt, wobei das in *vitro-Verfahren* die folgenden Schritte umfasst:

    (a) Bestimmen der Menge an hBNP in der Testprobe gemäß einem Immunoassay zur Quantifizierung der Menge an hBNP;
    (b) Bestimmen der Menge an menschlichem proBNP in der Probe;
    (c) Bestimmen des molaren Verhältnisses oder des Gewichtsverhältnisses von der Menge an menschlichem proBNP zu der Menge an hBNP in der Probe; und
    (d) Korrelieren des molaren Verhältnisses oder des Gewichtsverhältnisses mit dem Fortschreiten der Erkrankung in dem Subjekt, worin das Verhältnis niedriger verglichen mit dem in einer früheren Testprobe von dem Subjekt ist beim Fortschreiten, und das Verhältnis unverändert oder höher ist verglichen mit dem in einer früheren Testprobe von dem Subjekt beim nicht-Fortschreiten oder bei einer Verbesserung der kardiovaskulären Erkrankung;
    worin der Immunoassay zur Bestimmung der Menge an hBNP eine verminderte Kreuz-Reaktivität hat mit jeglichem menschlichem proBNP, das in der Testprobe vorhanden ist, und die folgenden Schritte umfasst:

    (I) In-Kontakt-bringen von mindestens einem Einfang-Antikörper, der an hBNP bindet, und der auf eine feste Phase immobilisiert wurde, um einen immobilisierten Antikörper zu erzeugen, mit der Testprobe, um eine erste Mischung zu bilden, die mindestens einen Einfang-Antikörper-hBNP-Komplex umfasst, worin der Einfang-Antikörper einen oder mehrere Antikörper mit einer Gleichgewichtsdissoziationskonstante ($K_D$) von zwischen ungefähr $3{,}0 \times 10^{-7}$ und ungefähr $1{,}0 \times 10^{-13}$ M umfasst;
    (II) In-Kontakt-bringen der ersten Mischung umfassend den mindestens einen Einfang-Antikörper-hBNP-Komplex mit mindestens einem Detektionsantikörper, der an hBNP bindet und der an einen detektierbaren Marker konjugiert wurde, um eine zweite Mischung zu bilden, die mindestens einen Einfang-Antikörper-hBNPmindestens ein Detektionsantikörper-Komplex umfasst, worin der Detektionsantikörper einen oder mehrere Antikörper mit einer Gleichgewichtsdissoziationskonstante ($K_D$) von zwischen ungefähr $3{,}0 \times 10^{-7}$ und ungefähr $1{,}0 \times 10^{-13}$ M umfasst; und
    (III) Bestimmen der Menge des mindestens einen Einfang-Antikörper-hBNP-mindestens ein Detektionsantikörper-Komplexes, der in Schritt (II) gebildet wurde, durch Detektieren des detektierbaren Markers als ein Maß für die Menge an hBNP, die in der Testprobe enthalten ist,

    worin der mindestens eine Einfang-Antikörper und der mindestens eine Detektionsantikörper, wenn sie zusammen verwendet werden, eine Kreuz-Reaktivität von weniger als ungefähr 20% zeigen mit jeglichem menschlichen proBNP, das in der Testprobe vorhanden ist.

4.  Das *in* vitro-Verfahren gemäß Anspruch 3, worin die Überwachung nach Behandlung der kardiovaskulären Erkrankung durchgeführt wird.

5. Ein *in vitro-Verfahren* zur Identifizierung eines Subjekts, welches profitieren würde von einer natriuretischen Peptidderivatbehandlung für die kardiovaskuläre Erkrankung, wobei das in *vitro*-Verfahren die folgenden Schritte umfasst:

(a) Bestimmen der Menge an menschlichem BNP in einer Testprobe von einem Subjekt, das ein oder mehrere klinische Indizien zeigt, die mit einer kardiovaskulären Erkrankung assoziiert sind, wobei die Bestimmung mit einem Immunoassay für die Quantifizierung der Menge an hBNP durchgeführt wird;
(b) Bestimmen der Menge an menschlichem proBNP in der Probe;
(c) Bestimmen des molaren Verhältnisses oder des Gewichtsverhältnisses von der Menge an menschlichem proBNP zu der Menge an hBNP in der Probe;
(d) Bestimmen, ob das molare Verhältnis oder das Gewichtsverhältnis, das in Schritt (c) bestimmt wurde, höher oder niedriger ist als ein vorbestimmter Level; und
(e) Ermitteln, ob das Subjekt von einer natriuretischen Peptidderivatbehandlung profitieren würde, basierend auf der Bestimmung in Schritt (d), worin, wenn das Verhältnis geringer ist verglichen mit dem vorbestimmten Level, das Subjekt als ein Subjekt ermittelt wird, das von einer natriuretischen Peptidderivatbehandlung nicht profitieren würde, und weiter worin, wenn das Verhältnis höher ist als ein vorbestimmter Level, dann das Subjekt als ein Subjekt ermittelt wird, das von einer natriuretischen Peptidderivatbehandlung profitieren würde;
worin der Immunoassay zur Bestimmung der Menge an menschlichem BNP eine verminderte Kreuz-Reaktivität hat mit jeglichem menschlichen proBNP, das in der Testprobe vorhanden ist, und die folgenden Schritte umfasst:

(I) In-Kontakt-bringen von mindestens einem Einfang-Antikörper, der an hBNP bindet, und der auf eine feste Phase immobilisiert wurde, um einen immobilisierten Antikörper zu erzeugen, mit der Testprobe, um eine erste Mischung zu bilden, die mindestens einen Einfang-Antikörper-hBNP-Komplex umfasst, worin der Einfang-Antikörper einen oder mehrere Antikörper mit einer Gleichgewichtsdissoziationskonstante ($K_D$) von zwischen ungefähr $3,0 \times 10^{-7}$ und ungefähr $1,0 \times 10^{-13}$ M umfasst;
(II) In-Kontakt-bringen der ersten Mischung umfassend den mindestens einen Einfang-Antikörper-hBNP-Komplex mit mindestens einem Detektionsantikörper, der an hBNP bindet und der an einen detektierbaren Marker konjugiert wurde, um eine zweite Mischung zu bilden, die mindestens einen Einfang-Antikörper-hBNPmindestens ein Detektionsantikörper-Komplex umfasst, worin der Detektionsantikörper einen oder mehrere Antikörper mit einer Gleichgewichtsdissoziationskonstante ($K_D$) von zwischen ungefähr $3,0 \times 10^{-7}$ und ungefähr $1,0 \times 10^{-13}$ M umfasst; und
(III) Bestimmen der Menge des mindestens einen Einfang-Antikörper-hBNP-mindestens ein Detektionsantikörper-Komplexes, der in Schritt (II) gebildet wurde, durch Detektieren des detektierbaren Markers als ein Maß für die Menge an hBNP, die in der Testprobe enthalten ist,

worin der mindestens eine Einfang-Antikörper und der mindestens eine Detektionsantikörper, wenn sie zusammen verwendet werden, eine Kreuz-Reaktivität von weniger als ungefähr 20% zeigen mit jeglichem menschlichen proBNP, das in der Testprobe vorhanden ist.

6. Das in *vitro*-verfahren gemäß Anspruch 5, worin das natriuretische Peptidderivat Nesiritid ist.

7. Ein *in vitro*-Verfahren zur Bestimmung, ob ein Subjekt an einer kardiovaskulären Komplikation gelitten hat als Ergebnis der Verabreichung von einer oder mehreren pharmazeutischen Zusammensetzungen an das Subjekt, wobei das *in vitro*-Verfahren die folgenden Schritte umfasst:

(a) Bestimmen der Menge an menschlichem BNP in einer Testprobe bevor dem Subjekt eine oder mehrere pharmazeutische Zusammensetzungen verabreicht wurden, mit einem Immunoassay zur Quantifizierung der Menge an hBNP, die in einer Testprobe vorhanden ist;
(b) Bestimmen der Menge an menschlichem proBNP in der Probe;
(c) Bestimmen des molaren Verhältnisses oder des Gewichtsverhältnisses von der Menge an menschlichem proBNP zu der Menge an hBNP in der Probe;
(d) Bestimmen der Menge an menschlichem BNP in einer zweiten Testprobe von dem Subjekt, nachdem dem Subjekt eine oder mehrere pharmazeutische Zusammensetzungen verabreicht wurden, wobei die Bestimmung mit dem Immunoassay durchgeführt wird;
(e) Bestimmen der Menge an menschlichem proBNP in der zweiten Testprobe;
(f) Bestimmen des molaren Verhältnisses oder des Gewichtsverhältnisses von der Menge an menschlichem proBNP zu der Menge an hBNP in der zweiten Testprobe; und

(g) Vergleichen des molaren Verhältnisses oder des Gewichtsverhältnisses, das in Schritt (c) bestimmt wurde, mit dem molaren Verhältnis oder dem Gewichtsverhältnis in Schritt (f), worin, wenn das molare Verhältnis oder das Gewichtsverhältnis, das in Schritt (c) bestimmt wurde, verglichen mit dem molaren Verhältnis oder dem Gewichtsverhältnis, das in Schritt (f) bestimmt wurde, unverändert ist, dann bestimmt wird, dass das Subjekt nicht an einer kardiovaskulären Komplikation gelitten hat als Ergebnis der Verabreichung von einer oder mehreren pharmazeutischen Zusammensetzungen, und weiter worin, wenn das molare Verhältnis oder das Gewichtsverhältnis, das in Schritt (c) bestimmt wurde, verglichen mit dem molaren Verhältnis oder dem Gewichtsverhältnis bestimmt in Schritt (f) verändert ist, dann bestimmt wird, dass das Subjekt an einer kardiovaskulären Komplikation gelitten hat als Ergebnis der Verabreichung von einer oder mehreren pharmazeutischen Zusammensetzungen;

worin der Immunoassay zur Bestimmung der Menge an menschlichem BNP eine verminderte Kreuz-Reaktivität mit jeglichem menschlichen proBNP hat, das in der Testprobe vorhanden ist, und die folgenden Schritt umfasst:

(I) In-Kontakt-bringen von mindestens einem Einfang-Antikörper, der an hBNP bindet, und der auf eine feste Phase immobilisiert wurde, um einen immobilisierten Antikörper zu erzeugen, mit der Testprobe, um eine erste Mischung zu bilden, die mindestens einen Einfang-Antikörper-hBNP-Komplex umfasst, worin der Einfang-Antikörper einen oder mehrere Antikörper mit einer Gleichgewichtsdissoziationskonstante ($K_D$) von zwischen ungefähr $3,0 \times 10^{-7}$ und ungefähr $1,0 \times 10^{-13}$ M umfasst;

(II) In-Kontakt-bringen der ersten Mischung umfassend den mindestens einen Einfang-Antikörper-hBNP-Komplex mit mindestens einem Detektionsantikörper, der an hBNP bindet und der an einen detektierbaren Marker konjugiert wurde, um eine zweite Mischung zu bilden, die mindestens einen Einfang-Antikörper-hBNPmindestens ein Detektionsantikörper-Komplex umfasst, worin der Detektionsantikörper einen oder mehrere Antikörper mit einer Gleichgewichtsdissoziationskonstante ($K_D$) von zwischen ungefähr $3,0 \times 10^{-7}$ und ungefähr $1,0 \times 10^{-13}$ M umfasst; und

(III) Bestimmen der Menge des mindestens einen Einfang-Antikörper-hBNP-mindestens ein Detektionsantikörper-Komplexes, der in Schritt (II) gebildet wurde, durch Detektieren des detektierbaren Markers als ein Maß für die Menge an hBNP, die in der Testprobe enthalten ist,

worin der mindestens eine Einfang-Antikörper und der mindestens eine Detektionsantikörper, wenn sie zusammen verwendet werden, eine Kreuz-Reaktivität von weniger als ungefähr 20% zeigen mit jeglichem menschlichen proBNP, das in der Testprobe vorhanden ist.

## Revendications

1. Procédé *In vitro* de détermination de la gravité d'une maladie cardiovasculaire chez un sujet, le procédé *in vitro* comprenant les étapes de :

(a) déterminer la quantité de hBNP dans un échantillon de test selon un immunotest pour quantifier la quantité de hBNP présente ;

(b) déterminer la quantité de proBNP humain dans ledit échantillon ;

(c) déterminer le rapport molaire ou le rapport massique de la quantité de proBNP humain à la quantité de hBNP dans ledit échantillon; et

(d) corréler le rapport molaire ou le rapport massique avec la gravité d'une maladie cardiovasculaire chez le sujet où, si le rapport est inférieur à un niveau prédéterminé, le sujet est déterminé comme ayant une gravité de maladie cardiovasculaire accrue, et si le rapport est supérieur à un niveau prédéterminé, le sujet est déterminé comme ayant une gravité de maladie cardiovasculaire réduite ;

où ledit immunotest pour déterminer la quantité de hBNP a une réactivité croisée réduite avec tout proBNP humain présent dans l'échantillon de test et comprend les étapes de :

(I) mettre en contact au moins un anticorps de capture qui se lie à hBNP et qui a été immobilisé sur une phase solide pour produire un anticorps immobilisé avec ledit échantillon de test pour former un premier mélange comprenant un complexe au moins un anticorps de capture-hBNP où ledit anticorps de capture comprend un ou plusieurs anticorps ayant une constante de dissociation à l'équilibre ($K_D$) entre environ $3,0 \times 10^{-7}$ et environ $1,0 \times 10^{-13}$ M;

(II) mettre en contact ledit premier mélange comprenant le complexe au moins un anticorps de capture-hBNP avec au moins un anticorps de détection qui se lie à hBNP et qui a été conjugué à un marqueur détectable pour former un second mélange comprenant un complexe au moins un anticorps de capture-hBNP-au moins un anticorps de détection, où l'anticorps de détection comprend un ou plusieurs anticorps ayant une constante de dissociation à l'équilibre ($K_D$) entre environ $3,0 \times 10^{-7}$ et environ $1,0 \times 10^{-13}$ M ; et

(III) déterminer la quantité du complexe au moins un anticorps de capture-hBNP-au moins un anticorps de détection formé dans l'étape (II) en détectant le marqueur détectable comme mesure de la quantité de hBNP contenue dans l'échantillon de test,

où le au moins un anticorps de capture et le au moins un anticorps de détection, quand ils sont utilisés ensemble, présentent une réactivité croisée inférieure à environ 20 % avec tout proBNP humain présent dans l'échantillon de test.

2. Procédé *in vitro* selon la revendication 1, où la maladie cardiovasculaire est choisie dans le groupe consistant en la maladie coronarienne, les maladies vasculaires périphériques, l'hypertension, l'infarctus du myocarde et l'insuffisance cardiaque.

3. Procédé *in vitro* de suivi de l'évolution d'une maladie cardiovasculaire chez un sujet, le procédé *in vitro* comprenant les étapes de :

   (a) déterminer la quantité de hBNP dans l'échantillon de test selon un immunotest pour quantifier la quantité de hBNP ;
   (b) déterminer la quantité de proBNP humain dans ledit échantillon ;
   (c) déterminer le rapport molaire ou le rapport massique de la quantité de proBNP humain à la quantité de hBNP dans ledit échantillon et
   (d) corréler le rapport molaire ou le rapport massique avec l'évolution de la maladie chez le sujet où le rapport est inférieur à celui dans un échantillon de test antérieur provenant du sujet ayant une évolution, et le rapport est inchangé ou supérieur à celui dans un échantillon de test antérieur provenant du sujet ayant une non-évolution ou une amélioration de la maladie cardiovasculaire ;
   où ledit immunotest pour déterminer la quantité de hBNP a une réactivité croisée réduite avec tout proBNP humain présent dans l'échantillon de test et comprend les étapes de :

   (I) mettre en contact au moins un anticorps de capture qui se lie à hBNP et qui a été immobilisé sur une phase solide pour produire un anticorps immobilisé avec ledit échantillon de test pour former un premier mélange comprenant un complexe au moins un anticorps de capture-hBNP où ledit anticorps de capture comprend un ou plusieurs anticorps ayant une constante de dissociation à l'équilibre ($K_D$) entre environ $3,0 \times 10^{-7}$ et environ $1,0 \times 10^{-13}$ M;
   (II) mettre en contact ledit premier mélange comprenant le complexe au moins un anticorps de capture-hBNP avec au moins un anticorps de détection qui se lie à hBNP et qui a été conjugué à un marqueur détectable pour former un second mélange comprenant un complexe au moins un anticorps de capture-hBNP-au moins un anticorps de détection, où l'anticorps de détection comprend un ou plusieurs anticorps ayant une constante de dissociation à l'équilibre ($K_D$) entre environ $3,0 \times 10^{-7}$ et environ $1,0 \times 10^{-13}$ M ; et
   (III) déterminer la quantité du complexe au moins un anticorps de capture-hBNP-au moins un anticorps de détection formé dans l'étape (II) en détectant le marqueur détectable comme mesure de la quantité de hBNP contenue dans l'échantillon de test,

   où le au moins un anticorps de capture et le au moins un anticorps de détection, quand ils sont utilisés ensemble, présentent une réactivité croisée inférieure à environ 20 % avec tout proBNP humain présent dans l'échantillon de test.

4. Procédé *in vitro* selon la revendication 3, où ledit suivi est réalisé après traitement pour la maladie cardiovasculaire.

5. Procédé *in vitro* d'identification d'un sujet qui bénéficierait d'un traitement avec un dérivé de peptide natriurétique pour une maladie cardiovasculaire, le procédé *in vitro* comprenant les étapes de :

   (a) déterminer la quantité de BNP humain dans un échantillon de test provenant d'un sujet qui présente un ou plusieurs indices cliniques associés avec une maladie cardiovasculaire, laquelle détermination est réalisée avec un immunotest pour quantifier la quantité de hBNP ;
   (b) déterminer la quantité de proBNP humain dans ledit échantillon ;
   (c) déterminer le rapport molaire ou le rapport massique de la quantité de proBNP humain à la quantité de hBNP dans ledit échantillon ;

(d) déterminer si le rapport molaire ou le rapport massique déterminé dans l'étape (c) est supérieur ou inférieur à un niveau prédéterminé ; et

(e) identifier si le sujet bénéficierait d'un traitement avec un dérivé de peptide natriurétique sur la base de la détermination dans l'étape (d), où, si le rapport est Inférieur au niveau prédéterminé, le sujet est identifié comme un sujet qui ne bénéficierait pas d'un traitement avec un dérivé de peptide natriurétique et en outre où, si le rapport est supérieur à un niveau prédéterminé, alors le sujet est identifié comme un sujet qui bénéficierait d'un traitement avec un dérivé de peptide natriurétlque ;

où ledit immunotest pour déterminer la quantité de BNP humain a une réactivité croisée réduite avec tout proBNP humain présent dans l'échantillon de test et comprend les étapes de :

(I) mettre en contact au moins un anticorps de capture qui se lie à hBNP et qui a été immobilisé sur une phase solide pour produire un anticorps immobilisé avec ledit échantillon de test pour former un premier mélange comprenant un complexe au moins un anticorps de capture-hBNP où ledit anticorps de capture comprend un ou plusieurs anticorps ayant une constante de dissociation à l'équilibre ($K_D$) entre environ $3,0 \times 10^{-7}$ et environ $1,0 \times 10^{-13}$ M ;

(II) mettre en contact ledit premier mélange comprenant le complexe au moins un anticorps de capture-hBNP avec au moins un anticorps de détection qui se lie à hBNP et qui a été conjugué à un marqueur détectable pour former un second mélange comprenant un complexe au moins un anticorps de capture-hBNP-au moins un anticorps de détection, où l'anticorps de détection comprend un ou plusieurs anticorps ayant une constante de dissociation à l'équilibre ($K_D$) entre environ $3,0 \times 10^{-7}$ et environ $1,0 \times 10^{-13}$ M ; et

(III) déterminer la quantité du complexe au moins un anticorps de capture-hBNP-au moins un anticorps de détection formé dans l'étape (II) en détectant le marqueur détectable comme mesure de la quantité de hBNP contenue dans l'échantillon de test,

où le au moins un anticorps de capture et le au moins un anticorps de détection, quand ils sont utilisés ensemble, présentent une réactivité croisée inférieure à environ 20 % avec tout proBNP humain présent dans l'échantillon de test.

**6.** Procédé *in vitro* selon la revendication 5, où le dérivé de peptide natriurétique est le nésiritide.

**7.** Procédé *in vitro* de détermination si un sujet a souffert d'une complication cardiovasculaire par suite de l'administration audit sujet d'une ou plusieurs compositions pharmaceutiques, le procédé *in vitro* comprenant les étapes de :

(a) déterminer la quantité de BNP humain dans un échantillon de test avant que le sujet se soit vu administrer une ou plusieurs compositions pharmaceutiques avec un immunotest pour quantifier la quantité de hBNP présente dans un échantillon de test ;

(b) déterminer la quantité de proBNP humain dans ledit échantillon;

(c) déterminer le rapport molaire ou le rapport massique de la quantité de proBNP humain à la quantité de hBNP dans ledit échantillon ;

(d) déterminer la quantité de BNP humain dans un second échantillon de test provenant du sujet après que le sujet se soit vu administrer une ou plusieurs compositions pharmaceutiques, laquelle détermination est réalisée avec ledit immunotest ;

(e) déterminer la quantité de proBNP humain dans ledit second échantillon de test ;

(f) déterminer le rapport molaire ou le rapport massique de la quantité de proBNP humain à la quantité de hBNP dans ledit second échantillon de test ; et

(g) comparer le rapport molaire ou le rapport massique déterminé dans l'étape (c) avec le rapport molaire ou massique dans l'étape (f), où, si le rapport molaire ou le rapport massique déterminé dans l'étape (c) est inchangé quand il est comparé au rapport molaire ou au rapport massique déterminé dans l'étape (f), alors le sujet est déterminé comme n'ayant pas souffert d'une complication cardiovasculaire par suite de l'administration d'une ou plusieurs compositions pharmaceutiques et en outre où, si le rapport molaire ou le rapport massique déterminé dans l'étape (c) est changé quand il est comparé au rapport molaire ou au rapport massique déterminé dans l'étape (f), alors le sujet est déterminé comme ayant souffert d'une complication cardiovasculaire par suite de l'administration d'une ou plusieurs compositions pharmaceutiques ;

où ledit immunotest pour déterminer la quantité de BNP humain a une réactivité croisée réduite avec tout proBNP humain présent dans l'échantillon de test et comprend les étapes de :

(I) mettre en contact au moins un anticorps de capture qui se lie à hBNP et qui a été Immobilisé sur une phase solide pour produire un anticorps immobilisé avec ledit échantillon de test pour former un premier mélange comprenant un complexe au moins un anticorps de capture-hBNP où ledit anticorps de capture comprend un ou plusieurs anticorps ayant une constante de dissociation à l'équilibre ($K_D$) entre environ $3{,}0 \times 10^{-7}$ et environ $1{,}0 \times 10^{-13}$ M ;

(II) mettre en contact ledit premier mélange comprenant le complexe au moins un anticorps de capture-hBNP avec au moins un anticorps de détection qui se lie à hBNP et qui a été conjugué à un marqueur détectable pour former un second mélange comprenant un complexe au moins un anticorps de capture-hBNP-au moins un anticorps de détection, où l'anticorps de détection comprend un ou plusieurs anticorps ayant une constante de dissociation à l'équilibre ($K_D$) entre environ $3{,}0 \times 10^{-7}$ et environ $1{,}0 \times 10^{-13}$ M ; et

(III) déterminer la quantité du complexe au moins un anticorps de capture-hBNP-au moins un anticorps de détection formé dans l'étape (II) en détectant le marqueur détectable comme mesure de la quantité de hBNP contenue dans l'échantillon de test,

où le au moins un anticorps de capture et le au moins un anticorps de détection, quand ils sont utilisés ensemble, présentent une réactivité croisée inférieure à environ 20 % avec tout proBNP humain présent dans l'échantillon de test.

Figure 1

Figure 2

Figure 3

Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6162902 A
- US 1595625 A
- US 74596307 A
- US 6677124 B
- US 20060183154 A
- US 20060172933 A
- US 20040266673 A
- US 5089424 A
- US 5006309 A
- US 5063081 A
- US 20030170881 A
- US 20040018577 A
- US 20050054078 A
- US 20060160164 A
- US 5468646 A
- US 20050014287 A

**Non-patent literature cited in the description**

- **YANDLE.** *J. Internal Med.,* 1994, vol. 235, 561-576
- **TATEYAMA et al.** *Biochem. Biophys. Res. Commun.,* 1992, vol. 185, 760-7
- **HUNT et al.** *Biochem. Biophys. Res. Commun.,* 1995, vol. 214, 1175-83
- **CATALIOTTI et al.** *Mayo Clin. Proc.,* 2001, vol. 76, 111-1119
- **LIANG F. et al.** *J. American College of Cardiology,* 2007, vol. 49 (10), 1071-1078
- **SCHELLENBERGER, U. et al.** *Archives of Biochemistry and Biophysics,* 2006, vol. 451, 160-166
- **HAMMERER-LERCHER A. et al.** *Clinical Chemistry,* 2008, vol. 54 (5), 858-865
- **SEFERIAN, K. et al.** *Clinical Chemistry,* 2008, vol. 54 (5), 866-873
- **LAM et al.** *Journal of American College of Cardiology,* 2007, vol. 49 (11), 1193-1202
- **POLAK ; VAN NOORDEN.** Introduction to Immunocytochemistry. Springer Verlag, 1997
- **HAUGLAND.** Handbook of Fluorescent Probes and Research Chemicals. 1996

SEQUENCE LISTING

<110> Moore, Jeff Shih, Jessie

<120> HUMAN B-TYPE NATRIURETIC PEPTIDE ASSAY HAVING REDUCED CROSS-REACTIVITY WITH OTHER PEPTIDE FORMS

<130> 8480WOO1

<160> 4

<170> Patent In version 3.4

<210> 1
<211> 134
<212> PRT
<213> Homo sapiens

<400> 1

```
Met Asp Pro Gln Thr Ala Pro Ser Arg Ala Leu Leu Leu Leu Leu Phe
1               5               10              15

Leu His Leu Ala Phe Leu Gly Gly Arg Ser His Pro Leu Gly Ser Pro
            20              25              30

Gly Ser Ala Ser Asp Leu Glu Thr Ser Gly Leu Gln Glu Gln Arg Asn
            35              40              45

His Leu Gln Gly Lys Leu Ser Glu Leu Gln Val Glu Gln Thr Ser Leu
    50              55              60

Glu Pro Leu Gln Glu Ser Pro Arg Pro Thr Gly Val Trp Lys Ser Arg
65              70              75              80

Glu Val Ala Thr Glu Gly Ile Arg Gly His Arg Lys Met Val Leu Tyr
            85              90              95

Thr Leu Arg Ala Pro Arg Ser Pro Lys Met Val Gln Gly Ser Gly Cys
            100             105             110

Phe Gly Arg Lys Met Asp Arg Ile Ser Ser Ser Ser Gly Leu Gly Cys
        115             120             125

Lys Val Leu Arg Arg His
    130
```

<210> 2
<211> 108
<212> PRT
<213> Homo sapiens

<400> 2

```
His Pro Leu Gly Ser Pro Gly Ser Ala Ser Asp Leu Glu Thr Ser Gly
```

```
     1                   5                    10                      15

Leu Gln Glu Gln Arg Asn His Leu Gln Gly Lys Leu Ser Glu Leu Gln
            20                  25                  30

Val Glu Gln Thr Ser Leu Glu Pro Leu Gln Glu Ser Pro Arg Pro Thr
            35                  40                  45

Gly Val Trp Lys Ser Arg Glu Val Ala Thr Glu Gly Ile Arg Gly His
        50                  55                  60

Arg Lys Met Val Leu Tyr Thr Leu Arg Ala Pro Arg Ser Pro Lys Met
65                  70                  75                      80

Val Gln Gly Ser Gly Cys Phe Gly Arg Lys Met Asp Arg Ile Ser Ser
            85                  90                  95

Ser Ser Gly Leu Gly Cys Lys Val Leu Arg Arg His
            100                 105
```

<210> 3
<211> 76
<212> PRT
<213> Homo sapiens

<400> 3

```
His Pro Leu Gly Ser Pro Gly Ser Ala Ser Asp Leu Glu Thr Ser Gly
1                   5                   10                  15

Leu Gln Glu Gln Arg Asn His Leu Gln Gly Lys Leu Ser Glu Leu Gln
            20                  25                  30

Val Glu Gln Thr Ser Leu Glu Pro Leu Gln Glu Ser Pro Arg Pro Thr
            35                  40                  45

Gly Val Trp Lys Ser Arg Glu Val Ala Thr Glu Gly Ile Arg Gly His
        50                  55                  60

Arg Lys Met Val Leu Tyr Thr Leu Arg Ala Pro Arg
65                  70                  75
```

<210> 4
<211> 32
<212> PRT
<213> Homo sapiens

<400> 4

```
Ser Pro Lys Met Val Gln Gly Ser Gly Cys Phe Gly Arg Lys Met Asp
1               5                   10                  15

Arg Ile Ser Ser Ser Ser Gly Leu Gly Cys Lys Val Leu Arg Arg His
          20                  25                  30
```